# EUROPEAN PATENT APPLICATION

(11) **EP 4 755 990 A1**
(43) Date of publication of application: **10.06.2026**
(21) Application number: 24849210.0
(22) Date of filing: 31.07.2024
(51) Int. Cl.: C09K 5/10

(54) **COMPOSITION FOR HEAT TRANSFER FLUIDS, DEVICE FOR HEAT TRANSFER, AND HEAT TRANSFER METHOD**

(30) Priority: 31.07.2023 JP 2023125027; 10.08.2023 JP 2023131645; 10.08.2023 JP 2023131650; 10.08.2023 JP 2023131651; 31.08.2023 JP 2023141588; 19.04.2024 JP 2024068659; 19.04.2024 JP 2024068660; 19.04.2024 JP 2024068661; 19.04.2024 JP 2024068663; 19.04.2024 JP 2024068667
(71) Applicant: Daikin Industries, Ltd., Osaka-shi, Osaka 530-0001 (JP)
(72) Inventor: MARI, Daichi, Osaka-Shi, Osaka 530-0001 (JP); SHIRAI, Atsushi, Osaka-Shi, Osaka 530-0001 (JP); SUZUKI, Yuki, Osaka-Shi, Osaka 530-0001 (JP); INOUE, Mayu, Osaka-Shi, Osaka 530-0001 (JP); KUROKI, Yoshichika, Osaka-Shi, Osaka 530-0001 (JP)
(74) Representative: Hoffmann Eitle
(86) International application number: PCT/JP2024/027360
(87) International publication number: WO 2025/028565

(57) **Abstract**

The present invention provides a composition for heat transfer fluid, the composition comprising:
a hexafluoropropene trimer represented by C₉F₁₈; and
(i) CₘF₂ₘ and/or CₙF₍₂ₙ₋₂₎ where m is an integer of 4 to 12 except 9 and n is an integer of 4 to 12, (ii) water where a content of the water relative to a total amount of the hexafluoropropene trimer represented by C₉F₁₈ regarded as 100 parts by mass is from 0.0001 to 0.1 parts by mass, and/or (iii) fluoride ions where a content of the fluoride ions relative to the total amount of the hexafluoropropene trimer represented by C₉F₁₈ regarded as 100 parts by mass is from 0.0000001 to 5 parts by mass.

## Description

### TECHNICAL FIELD

The present disclosure relates to a composition for heat transfer fluid, a heat transfer apparatus, and a heat transfer method.

### BACKGROUND ART

Hexafluoropropene (HFP) trimers are known to be usable in the form of a composition for heat transfer fluid (PTL 1).

HFP trimers have low global warming potential (GWP) and low toxicity, and therefore have been receiving attention as an alternative to chlorofluorocarbon (CFC) and hydrochlorofluorocarbon (HCFC).

### CITATION LIST

### PATENT LITERATURE

PTL 1: WO2018/172919

### SUMMARY OF INVENTION

### TECHNICAL PROBLEM

In light of the above-described circumstances, the present disclosure aims at providing a composition for heat transfer fluid, as a novel mixture.

### SOLUTION TO PROBLEM

The inventors of the present invention have conducted intensive research to achieve the above-mentioned objective, and as a result, have found that by mixing a certain fluorocarbon with an HFP trimer, a certain amount of water, and/or a certain amount of fluoride ions, it is possible to provide a composition for heat transfer fluid as a novel mixture.

More specifically, the present disclosure encompasses below-described aspects.

[Aspect 1] A composition for heat transfer fluid, the composition comprising:
a hexafluoropropene trimer represented by C₉F₁₈; and
(i) CₘF₂ₘ and/or CₙF₍₂ₙ₋₂₎ where m is an integer of 4 to 12 except 9 and n is an integer of 4 to 12, (ii) water where a content of the water relative to a total amount of the hexafluoropropene trimer represented by C₉F₁₈ regarded as 100 parts by mass is from 0.0001 to 0.1 parts by mass, and/or (iii) fluoride ions where a content of the fluoride ions relative to the total amount of the hexafluoropropene trimer represented by C₉F₁₈ regarded as 100 parts by mass is from 0.0000001 to 5 parts by mass.

[Aspect 2] The composition for heat transfer fluid according to Aspect 1, wherein the hexafluoropropene trimer includes at least one selected from the group consisting of compounds represented by formulae (I) to (III) below.

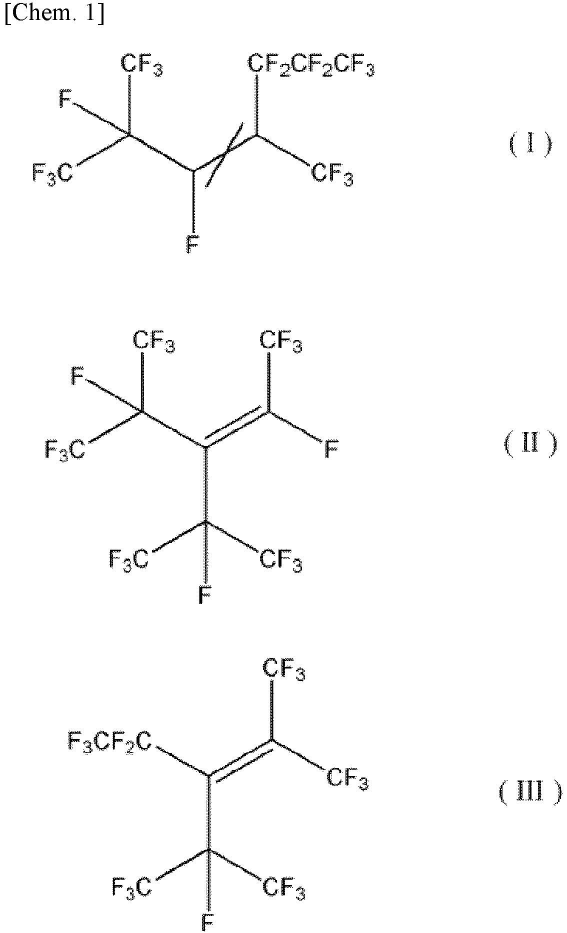

[Aspect 3] The composition for heat transfer fluid according to Aspect 2, wherein a content of the compound represented by the formula (I) relative to the total amount of the hexafluoropropene trimer is 85 mass% or more.

[Aspect 4] The composition for heat transfer fluid according to Aspect 2, wherein a content of the compound represented by the formula (I) relative to the total amount of the hexafluoropropene trimer is less than 85 mass%.

[Aspect 5] The composition for heat transfer fluid according to Aspect 2, wherein a content of the compound represented by the formula (I) relative to the total amount of the hexafluoropropene trimer is not less than 50 mass% and less than 85 mass%.

[Aspect 6] The composition for heat transfer fluid according to Aspect 1, comprising the hexafluoropropene trimer represented by C₉F₁₈ and (i) CₘF₂ₘ and/or CₙF₍₂ₙ₋₂₎ where m is an integer of 4 to 12 except 9 and n is an integer of 4 to 12.

[Aspect 7] The composition for heat transfer fluid according to Aspect 1, wherein m is an integer of 6 to 11 except 9 and n is an integer of 6 to 11.

[Aspect 8] The composition for heat transfer fluid according to Aspect 1, wherein a content of CₘF₂ₘ and/or CₙF₍₂ₙ₋₂₎ relative to the total amount of the hexafluoropropene trimer represented by C₉F₁₈ regarded as 100 parts by mass is from 0.0001 to 10 parts by mass.

[Aspect 9] The composition for heat transfer fluid according to Aspect 1, comprising the hexafluoropropene trimer represented by C₉F₁₈ and (ii) water.

[Aspect 10] The composition for heat transfer fluid according to Aspect 1, comprising the hexafluoropropene trimer represented by C₉F₁₈ and (iii) fluoride ions.

[Aspect 11] The composition for heat transfer fluid according to Aspect 1, further comprising a hexafluoropropene tetramer represented by C₁₂F₂₄, wherein a content of the hexafluoropropene trimer relative to a total amount of the hexafluoropropene trimer and the hexafluoropropene tetramer is 80 mass% or more.

[Aspect 12] The composition for heat transfer fluid according to Aspect 11, wherein the content of the hexafluoropropene trimer relative to the total amount of the hexafluoropropene trimer and the hexafluoropropene tetramer is from 90 mass% to 99.99 mass%.

[Aspect 13] The composition for heat transfer fluid according to Aspect 11, wherein the hexafluoropropene tetramer includes 1,1,1,2,5,6,6,6-octafluoro-2,3,5-tris(trifluoromethyl)-4-(perfluoropropyl-2-yl)-3-hexene.

[Aspect 14] The composition for heat transfer fluid according to Aspect 1, further comprising a conductive substance, wherein a content of the conductive substance is 100 ppm by mass or less.

[Aspect 15] The composition for heat transfer fluid according to Aspect 1, further comprising a conductive substance, wherein a content of insoluble matter of 5 µm or more is 10 pieces/mL or less.

[Aspect 16] A heat transfer fluid comprising the composition for heat transfer fluid according to Aspect 1 or 2.

[Aspect 17] Use of the composition for heat transfer fluid according to Aspect 1 or 2 as a heat transfer fluid.

[Aspect 18] A heat transfer apparatus comprising:
a device; and
a mechanism for transferring heat to the device or from the device, wherein the mechanism comprises the composition for heat transfer fluid according to Aspect 1 or 2.

[Aspect 19] A heat transfer method comprising:
preparing a device; and
transferring heat to the device or from the device with the use of the composition for heat transfer fluid according to Aspect 1 or 2.

### ADVANTAGEOUS EFFECTS OF INVENTION

The above-described composition according to the present disclosure allows for providing a composition for heat transfer fluid, as a novel mixture.

### DESCRIPTION OF EMBODIMENTS

Herein, the term "contain" conceptually encompasses "comprise", "consist essentially of", and "consist of". Moreover, herein, a numerical range expressed as "(from) A to B" means not less than A and not more than B.

### (1. Composition for Heat Transfer Fluid)

A composition for heat transfer fluid according to the present disclosure comprises (i) CₘF₂ₘ and/or CₙF₍₂ₙ₋₂₎ [where m is an integer of 4 to 12 except 9 and n is an integer of 4 to 12], (ii) water (a content of the water relative to a total amount of the hexafluoropropene trimer represented by C₉F₁₈ regarded as 100 parts by mass is from 0.0001 to 0.1 parts by mass), and/or (iii) fluoride ions (a content of the fluoride ions relative to the total amount of the hexafluoropropene trimer represented by C₉F₁₈ regarded as 100 parts by mass is from 0.0000001 to 5 parts by mass).

The hexafluoropropene trimer is not limited and may be selected from a wide variety of known ones represented by C₉F₁₈.

Specifically, the hexafluoropropene trimer may be a trimer including at least one selected from the group consisting of compounds represented by formulae (I) to (III) below.

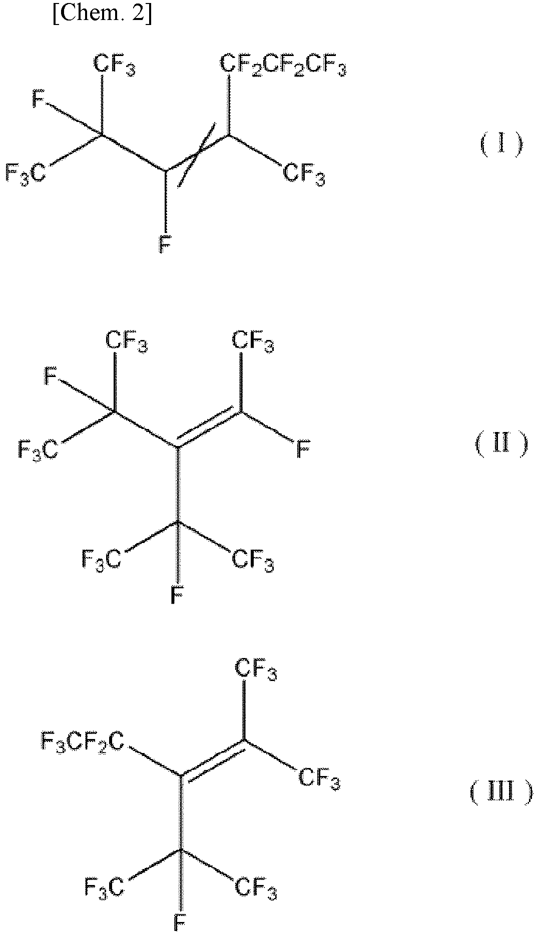

Herein, the compound represented by the formula (I) includes both E- and Z-diastereomers unless otherwise specified.

As the hexafluoropropene trimer contained in the composition for heat transfer fluid according to the present disclosure, only one of the compounds represented by the formulae (I) to (III) may be contained, or a mixture of two or three of them may be contained.

The proportion of the compound represented by the formula (I) in the total amount of the HFP trimer (namely, the total amount of the compounds represented by the formulae (I), (II), and (III)) is preferably 1 mass% or more, more preferably 10 mass% or more, further preferably 30 mass% or more, further more preferably 40 mass% or more, particularly preferably 45 mass% or more, particularly preferably 50 mass% or more. In some embodiments, the compound represented by the formula (I) in the total amount of the HFP trimer may be 85 mass% or more. In this case, the HFP trimer mixture has a low viscosity. Moreover, the compound represented by the formula (I) in the total amount of the HFP trimer is preferably 99 mass% or less, more preferably 90 mass% or less, further preferably 85 mass% or less (or less than 85 mass%), yet further preferably 80 mass% or less, particularly preferably 70 mass% or less, most preferably 60 mass% or less. For example, the proportion of the compound represented by the formula (I) in the total amount of the HFP trimer may be from 10 mass% to 90 mass%, or from 30 mass% to 90 mass%, or from 10 mass% to 85 mass%, or from 35 mass% to 85 mass%, or not less than 10 mass% and less than 85 mass%, or not less than 20 mass% and less than 85 mass%, or not less than 30 mass% and less than 85 mass%, or not less than 40 mass% and less than 85 mass%, or not less than 50 mass% and less than 85 mass%, or from 40 mass% to 80 mass%, or from 55 mass% to 80 mass%, or from 60 mass% to 75 mass%, or from 65 mass% to 70 mass%, or from 35 mass% to 60 mass%, or from 50 mass% to 60 mass%, and it may be preferably from 30 mass% to 90 mass%, preferably not less than 40 mass% and less than 85 mass%, more preferably from 40 mass% to 80 mass%, further preferably from 45 mass% to 70 mass%, further more preferably from 50 mass% to 60 mass%.

Similarly, the proportion of the compound represented by the formula (II) in the total amount of the HFP trimer is preferably 1 mass% or more, more preferably 5 mass% or more, particularly preferably 10 mass% or more. Moreover, the compound represented by the formula (II) in the total amount of the HFP trimer is 70 mass% or less, more preferably 50 mass% or less, yet further preferably 30 mass% or less, particularly preferably 20 mass% or less.

Similarly, the proportion of the compound represented by the formula (III) in the total amount of the HFP trimer is preferably 1 mass% or more, more preferably 5 mass% or more, particularly preferably 10 mass% or more. Moreover, the compound represented by the formula (III) in the total amount of the HFP trimer regarded as 100 mass% is preferably 70 mass% or less, more preferably 50 mass% or less, yet further preferably 30 mass% or less, particularly preferably 20 mass% or less.

The mass ratio between the compound represented by the formula (II) and the compound represented by the formula (III) in the composition for heat transfer fluid according to the present disclosure is not limited, and, for example, it may be from 1:9 to 9: 1, or from 2:8 to 8:2, or from 3:7 to 7:3, or from 4:6 to 6:4, or from 4.5:5.5 to 5.5:4.5.

The compounds represented by the formulae (I) to (III) may be produced by a conventional procedure, and examples of the procedure include, but not limited to, a method described in WO2018/172919. Moreover, the compounds may be obtained by trimerization of HFPs, but this is not limitative and the compounds may be obtained by a method selected from a wide range of methods.

The composition for heat transfer fluid according to the present disclosure may contain a hexafluoropropene trimer represented by C₉F₁₈ other than the compounds represented by the formulae (I) to (III).

The composition for heat transfer fluid according to the present disclosure may contain a hexafluoropropene dimer.

The hexafluoropropene dimer may include (E)-1,1,1,2,3,4,5,5,5-nonafluoro-4-(trifluoromethyl)-2-pentene, (Z)-1,1,1,2,3,4,5,5,5-nonafluoro-4-(trifluoromethyl)-2-pentene, or 1,1,3,4,4,5,5-nonafluoro-2-(trifluoromethyl)-2-pentene.

In one aspect, the composition for heat transfer fluid according to the present disclosure may contain a hexafluoropropene tetramer.

The hexafluoropropene tetramer may include 1,1,1,2,5,6,6,6-octafluoro-2,3,5-tris(trifluoromethyl)-4-(perfluoropropyl-2-yl)-3-hexene.

In the composition for heat transfer fluid according to the present disclosure, the content of the hexafluoropropene trimer relative to the total amount of the hexafluoropropene trimer and the hexafluoropropene tetramer may be 80 mass% or more, preferably 85 mass% or more, more preferably 90 mass% or more, and, for example, it may be 95 mass% or more, or 98 mass% or more, or 99 mass% or more, or 99.9 mass% or more.

In the composition for heat transfer fluid according to the present disclosure, the content of the hexafluoropropene trimer relative to the total amount of the hexafluoropropene trimer and the hexafluoropropene tetramer may be preferably 99.999 mass% or less, more preferably 99.99 mass% or less, and, for example, it may be 99.9 mass% or less, or 99 mass% or less, or 95 mass% or less, or 90 mass% or less, or 85 mass% or less.

In the composition for heat transfer fluid according to the present disclosure, the content of the hexafluoropropene trimer relative to the total amount of the hexafluoropropene trimer and the hexafluoropropene tetramer may be preferably from 80 mass% to 99.999 mass%, more preferably from 85 mass% to 99.99 mass%, and, for example, it may be from 90 mass% to 99.99 mass%, or from 95 mass% to 99.99 mass%, or from 99 mass% to 99.99 mass%, or from 99 mass% to 99.9 mass%.

The total amount of the hexafluoropropene trimer and the hexafluoropropene tetramer in the composition for heat transfer fluid may be preferably 80 mass% or more, more preferably 85 mass% or more, further preferably 90 mass% or more, further more preferably 95 mass% or more, and, for example, it may be 98 mass% or more, or 99 mass% or more, or 99.9 mass% or more. The total amount of the hexafluoropropene trimer and the hexafluoropropene tetramer in the composition for heat transfer fluid may be substantially 100 mass%. In other words, the composition for heat transfer fluid according to the present disclosure may be a mixture of the hexafluoropropene trimer and the hexafluoropropene tetramer.

### (1-1. CₘF₂ₘ and/or CₙF₍₂ₙ₋₂₎)

In one aspect, the composition for heat transfer fluid according to the present disclosure may contain the hexafluoropropene (HFP) trimer represented by C₉F₁₈ as well as CₘF₂ₘ and/or CₙF₍₂ₙ₋₂₎ [where m is an integer of 4 to 12 except 9 and n is an integer of 4 to 12].

m is an integer of 4 or more, preferably an integer of 5 or more, more preferably an integer of 6 or more. Moreover, n is an integer of 12 or less, preferably an integer of 11 or less, more preferably an integer of 10 or less. It should be noted that m does not include 9. Particularly preferably, m is 8.

n is an integer of 4 or more, preferably an integer of 5 or more, more preferably an integer of 6 or more. Moreover, n is an integer of 12 or less, preferably an integer of 11 or less, more preferably an integer of 10 or less. Particularly preferably, n is 9.

CₘF₂ₘ may be either a chain compound or a cyclic compound that may have a substituted structure. The chain compound may be a so-called alkene, and it may be either linear or branched.

CₙF₍₂ₙ₋₂₎ may be either a chain compound or a cyclic compound that may have a substituted structure. The chain compound may be a so-called diene or alkyne, and it may be either linear or branched.

The present disclosure is based on a surprising finding that a composition containing an HFP trimer and CₘF₂ₘ and/or CₙF₍₂ₙ₋₂₎ functions as a heat transfer fluid. In addition, when the composition for heat transfer fluid according to the present disclosure contains CₘF₂ₘ and/or CₙF₍₂ₙ₋₂₎, the stability of the HFP trimer is enhanced.

Preferably, the content of CₘF₂ₘ and/or CₙF₍₂ₙ₋₂₎ in the entire composition for heat transfer fluid according to the present disclosure is 0.0001 mass% or more.

Moreover, the content of CₘF₂ₘ and/or CₙF₍₂ₙ₋₂₎ in the entire composition for heat transfer fluid according to the present disclosure is preferably 10 mass% or less, preferably 5 mass% or less, further preferably 1 mass% or less.

In one aspect, in the composition for heat transfer fluid, the content of CₘF₂ₘ and/or CₙF₍₂ₙ₋₂₎ relative to the total amount of the HFP trimer regarded as 100 parts by mass is preferably 0.0001 parts by mass or more, more preferably 0.01 parts by mass or more, further preferably 0.1 parts by mass or more.

Moreover, in the composition for heat transfer fluid, the content of CₘF₂ₘ and/or CₙF₍₂ₙ₋₂₎ relative to the total amount of the HFP trimer regarded as 100 parts by mass is preferably 10 parts by mass or less, more preferably 5 parts by mass or less, further preferably 1 part by mass or less.

When multiple types of CₘF₂ₘ and/or CₙF₍₂ₙ₋₂₎ are contained, the above-mentioned content means the total amount of them.

When the content of CₘF₂ₘ and/or CₙF₍₂ₙ₋₂₎ falls within the above-mentioned range, degradation of the HFP trimer represented by C₉F₁₈ can be reduced and thereby an increase of fluoride ions and a rise of acidity can be reduced.

### (1-2. Water)

In one aspect, the composition for heat transfer fluid according to the present disclosure may contain water. The content of water in the composition for heat transfer fluid may be 1 ppm by mass or more, and preferably, it is 5 ppm by mass or more. When the content of water is set to a certain level or more, such as 1 ppm by mass or more, for example, electrification of the composition for heat transfer fluid that can occur when the composition for heat transfer fluid becomes less stable can be reduced. Moreover, the content of water in the composition for heat transfer fluid is 1000 ppm by mass or less, preferably 500 ppm by mass or less, more preferably 100 ppm by mass or less, yet further preferably 20 ppm by mass or less. When the content of water is set to a certain level or less, such as 1000 ppm by mass or less, for example, degradation of the HFP trimer represented by C₉F₁₈ during heating can be reduced and thereby an excessive increase of fluoride ions and a rise of acidity can be reduced.

In one aspect, the content of water relative to the total amount of the compound represented by C₉F₁₈ regarded as 100 parts by mass is preferably 0.0001 parts by mass or more, more preferably 0.0005 parts by mass or more, further preferably 0.001 parts by mass or more.

Moreover, the content of water relative to the total amount of the compound represented by C₉F₁₈ regarded as 100 parts by mass is preferably 10 parts by mass or less, more preferably 5 parts by mass or less, further preferably 1 part by mass or less.

When the composition for heat transfer fluid according to the present disclosure contains a certain amount of water, dielectric strength is enhanced. The dielectric strength of the composition for heat transfer fluid according to the present disclosure may be preferably 40 kV or more, preferably 50 kV or more, further preferably 60 kV or more.

### (1-3. Fluoride Ions)

In one aspect, the composition for heat transfer fluid according to the present disclosure may contain fluoride ions in a content of 0.0000001 mass% or more relative to the entire composition. The amount of fluoride ions in the entire composition is preferably 0.000001 mass% or more, more preferably 0.0001 mass% or more, further preferably 0.001 mass% or more. When the concentration of fluoride ions is 0.0000001 mass% or more, stability of the composition for heat transfer fluid can be maintained and electrification of the composition can be reduced.

Moreover, the amount of fluoride ions contained in the composition for heat transfer fluid according to the present disclosure is 5 mass% or less, preferably 1 mass% or less, more preferably 0.1 mass% or less relative to the entire composition. When the amount of fluoride ions is 5 mass% or less, degradation of the compound represented by C₉F₁₈ during heating can be reduced.

In one aspect, the amount of fluoride ions relative to the total amount of the compound represented by C₉F₁₈ regarded as 100 parts by mass is preferably 0.0000001 parts by mass or more, more preferably 0.000001 parts by mass or more, more preferably 0.0001 parts by mass or more, further preferably 0.001 parts by mass or more.

Moreover, the amount of fluoride ions relative to the total amount of the compound represented by C₉F₁₈ regarded as 100 parts by mass is preferably 5 parts by mass or less, more preferably 1 part by mass or less, further preferably 0.1 parts by mass or less.

The amount of fluoride ions can be measured in the following manner, for example. To a specimen, an equal amount (weight) of distilled water is added, and the resultant is shaken for about 20 seconds to extract F ions into the aqueous layer. Then, 2.5 to 3.0 mL of the aqueous layer is taken out with a pipette and mixed with twice the amount of TISAB solution (total ionic strength adjustment buffer), and the resulting liquid is used as a sample for measurement with a fluoride ion meter.

The source of the fluoride ions is not limited and may be selected from a wide range of known fluoride ion sources. Specific examples include ions from hydrogen fluoride, sodium fluoride, sodium hydrogen fluoride, potassium fluoride, potassium hydrogen fluoride, lithium fluoride, cesium fluoride, calcium fluoride, magnesium fluoride, aluminum fluoride, zinc fluoride, silver fluoride, and iron fluoride. The source of the fluoride ions may include only one type of them, or may include a plurality of types of them. Preferably, the source of the fluoride ions is hydrogen fluoride.

### (1-4. Other Components)

The composition for heat transfer fluid according to the present disclosure may contain perfluorotripropylamine, in addition to the hexafluoropropene trimer. The composition for heat transfer fluid containing the hexafluoropropene trimer and perfluorotripropylamine may be a quasi-azeotropic liquid. In the case where the composition for heat transfer fluid is a quasi-azeotropic liquid, the composition for heat transfer fluid does not undergo significant compositional changes even when it is vaporized, and therefore it has enhanced handleability.

Herein, the quasi-azeotropic liquid means a liquid where the molar fraction of each of the components of the quasi-azeotropic liquid in the gas phase differs by 10% or less from that in the liquid phase.

The perfluorotripropylamine is also referred to as tris(heptafluoropropyl)amine or N,N-bis(heptafluoropropyl)(heptafluoropropyl)amine, and represented by the general formula N(CF₂CF₂CF₃)ₐ(CF(CF₃)CF₃)₃₋ₐ (where a is an integer of 0 to 3). The perfluorotripropylamine may include only one type of compounds represented by the above general formula, or may include a plurality of types of them. N(CF₂CF₂CF₃)₃ is preferable, and N(CF₂CF₂CF₃)ₐ(CF(CF₃)CF₃)₃₋ₐ (where a is an integer of 0 to 2) may be included as impurities. Specific examples include a product under the trade name "Fluorinert (registered trademark)" (manufactured by 3M) (FC-3283) and the like.

The composition for heat transfer fluid according to the present disclosure may further contain perfluoropolyether, a methoxytridecafluoroheptene isomer mixture, perfluorotributylamine, and the like.

The perfluoropolyether is preferably represented by the general formula:

RO-Rf¹-R'

where
R and R' are the same as or different from each other and each is a monovalent group represented by -CₘF₂ₘ₊₁, in which m is an integer of 1 to 8, and
Rf¹ is a divalent fluoropolyoxyalkylene group containing 2 to 20 repeating units, in which the repeating unit is represented by:
   (i) -CFXO- (where X is F or CF₃);
   (ii) -CF₂CFXO- (where X is F or CF₃);
   (iii) -CFXCF₂O- (where X is F or CF₃);
   (iv) -CF₂CF₂CF₂O-; or
   (v) -CF₂CF₂CF₂CF₂O-, or
Rf¹ is a divalent group represented by:
   (vi) -(CF₂)ₙ-CFY-O- (where n is an integer of 0 to 3, Y is a monovalent group represented by the general formula -ORf²Z, Rf² is a divalent fluoropolyoxyalkylene group containing 2 to 20 repeating units represented by-CFXO-, -CF₂CFXO-, -CF₂CF₂CF₂O-, or -CF₂CF₂CF₂CF₂O-, Xs are the same as or different from each other and each is F or CF₃, and Z is a monovalent C₁₋₅ perfluoroalkyl group).

Specific examples of the perfluoropolyether include products under the trade names GALDEN (registered trademark) "HT135" and GALDEN (registered trademark) "HT110" (both manufactured by Solvay), and the like.

Specifically, the methoxytridecafluoroheptene isomer mixture includes methyl-perfluoroheptene ether (MPHE) (C₇F₁₃OCH₃). Specific examples include a product under the trade name "Opteon SF10" (manufactured by Chemours) and the like.

When the composition for heat transfer fluid according to the present disclosure contains perfluorotripropylamine, perfluoropolyether, and a methoxytridecafluoroheptene isomer mixture, the content ratio of these components does not substantially affect the heat transfer fluid properties of the entire composition for heat transfer fluid because these components have similar heat transfer fluid properties to those of the compound represented by C₉F₁₈. Therefore, in this case, the composition for heat transfer fluid according to the present disclosure preferably contains the compound represented by C₉F₁₈ in 40 mass% to 99.9 mass%, more preferably 60 mass% to 99.9 mass%, further preferably 80 mass% to 99.9 mass%, relative to the entire composition for heat transfer fluid.

### (1-5. Impurities)

The composition for heat transfer fluid according to the present disclosure may contain conductive substances. The content of conductive substances is 100 ppm by mass or less, preferably 75 ppm by mass or less, more preferably 50 ppm by mass or less, further preferably 10 ppm by mass or less. Specific types of conductive substances that can enter into the device through a gap to cause a short circuit are metals and metal ions, and therefore, as needed, study and analysis on the content of conductive substances according to the present disclosure can be performed in terms of "at least one of metals and metal ions".

In another aspect, in the composition for heat transfer fluid according to the present disclosure, the content of insoluble matter of 5 µm or more (including solid matter such as resin fragments of the container and dirt and dust from the air, both conductive and not conductive) is preferably 10 pieces/mL or less, more preferably 5 pieces/mL or less, further preferably 3 pieces/mL or less.

### (1-6. Method of Producing Composition for Heat Transfer Fluid with Reduced Impurities)

The present disclosure provides a method of producing a composition for heat transfer fluid with reduced impurities. The method of producing a composition for heat transfer fluid according to the present disclosure has a step to perform purification treatment of a composition containing both a heat transfer fluid compound and conductive substances to obtain the composition for heat transfer fluid from which the conductive substances are reduced, characterized in that:
(1) the heat transfer fluid compound contains at least one selected from the group consisting of a compound represented by C₉F₁₈, a perfluoroalkene ether, and a perfluoropolyether; and
(2) the purification treatment is a treatment performed with the use of at least one selected from the group consisting of filtration filter, ion-exchange resin, metal-ion removal filter, metal-ion removing agent, distillation, fractionation, centrifugation, and electrostatic attraction.

In the present disclosure, "reducing (reduced)" in the context of purification means reducing the content ratio of conductive substances in the composition.

According to the research conducted by the inventors of the present disclosure, a conventionally known so-called heat transfer fluid contains conductive substances (such as metals, metal ions, carbon, conductive polymers, superconducting ceramic, and the like). The conductive substances include those inevitably contaminating due to the process of production of the heat transfer fluid and also those contaminating during use after production. In the case where the heat transfer fluid is used for transferring heat to and/or from a device, the conductive substances can enter into the device through a gap to cause a short circuit. Moreover, they can cause clogging of the piping through which the heat transfer fluid is circulated, during repeated use of the heat transfer fluid. A conventionally known heat transfer fluid, because it contains conductive substances, can also be expressed as "a composition containing both a heat transfer fluid and conductive substances". The method of producing a composition for heat transfer fluid according to the present disclosure is characterized in that it performs a specific purification treatment of a composition containing both a heat transfer fluid and conductive substances (hereinafter also called "a pre-purification composition") to obtain the composition for heat transfer fluid from which the conductive substances are reduced.

### (1-6-1) Composition Containing Both Heat Transfer Fluid Compound and Conductive Substances (Pre-Purification Composition)

The pre-purification composition may contain both a heat transfer fluid compound and conductive substances. Here, as the heat transfer fluid compound, at least one selected from the group consisting of a compound represented by C₉F₁₈, a perfluoroalkene ether, and a perfluoropolyether is contained.

### (1-6-2) Compound Represented by C₉F₁₈

The compound represented by C₉F₁₈ is the above-mentioned hexafluoropropene trimer.

### (1-6-3) Perfluoroalkene Ether

Preferably, the perfluoroalkene ether may be selected from a wide variety of known compounds represented by the formula (V) below:

C₇F₁₃OR (V)

[where R is Me or Et].

Examples of the perfluoroalkene ether represented by the formula (V) include at least one unsaturated fluoroether selected from the group consisting of CF₃(CF₂)ₓCF=CFCF(OR)(CF₂)_{y}CF₃, CF₃(CF₂)ₓC(OR)=CFCF₂(CF₂)_{y}CF₃, CF₃CF=CFCF(OR)(CF₂)ₓ(CF₂)_{y}CF₃, and CF₃(CF₂)ₓCF=C(OR)CF₂(CF₂)_{y}CF₃ [where R is Me or Et, x and y are independently 0, 1, 2, or 3, and x+y=1, 2, or 3].

Specific examples of the perfluoroalkene ether represented by the formula (V) include 5-methoxyperfluoro-3-heptene, 3-methoxyperfluoro-3-heptene, 4-methoxyperfluoro-2-heptene, 3-methoxyperfluoro-2-heptene, 4-methoxyperfluoro-2-pentene, 2-methoxyperfluoro-2-pentene, 3-methoxyperfluoro-2-pentene, 2-methoxyperfluoro-3-pentene, cis- and trans-2-methoxyperfluoro-2-octene, 2-methoxyperfluoro-3-octene, and the like.

A preferable structure of the perfluoroalkene ether represented by the formula (V) is methylperfluoroheptene ether. The methylperfluoroheptene ether may include a mixture of two or more structural and/or stereoisomers. A specific example of the methylperfluoroheptene ether is a mixture of about 48 to about 52 mass% 5-methoxyperfluoro-3-heptene, about 18 to about 22 mass% 3-methoxyperfluoro-3-heptene, about 18 to about 22 mass% 4-methoxyperfluoro-2-heptene, and about 6 to about 10 mass% 4-methoxyperfluoro-3-heptene.

Specifically, the perfluoroalkene ether includes methyl-perfluoroheptene ether (MPHE) (C₇F₁₃OCH₃). Specific examples include a product under the trade name "Opteon SF10" (manufactured by Chemours) and the like.

### (1-6-4) Perfluoropolyether

The perfluoropolyether (PFPE) is preferably represented by the formula (IV) below:

RO-Rf¹-R' (IV)

where
R and R' are the same as or different from each other and each is a monovalent group represented by -CₘF₂ₘ₊₁, in which m is an integer of 1 to 8, and
Rf¹ is a divalent fluoropolyoxyalkylene group containing 2 to 20 repeating units, in which the repeating unit is represented by:
   (i) -CFXO- (where X is F or CF₃);
   (ii) -CF₂CFXO- (where X is F or CF₃);
   (iii) -CFXCF₂O- (where X is F or CF₃);
   (iv) -CF₂CF₂CF₂O-; or
   (v) -CF₂CF₂CF₂CF₂O-, or
Rf¹ is a divalent group represented by:
   (vi) -(CF₂)ₙ-CFY-O- (where n is an integer of 0 to 3, Y is a monovalent group represented by the general formula -ORf²Z, Rf² is a divalent fluoropolyoxyalkylene group containing 2 to 20 repeating units represented by-CFXO-, -CF₂CFXO-, -CF₂CF₂CF₂O-, or -CF₂CF₂CF₂CF₂O-, Xs are the same as or different from each other and each is F or CF₃, and Z is a monovalent C₁₋₅ perfluoroalkyl group). Here, the PFPE represented by the formula (IV) may be selected from a wide variety of known ones.

In a preferable structure of the PFPE represented by the formula (IV), m is an integer of 1 to 3 and Rf is selected from the below-listed ones:

(1) -(CF₂O)ₐ-(CF₂CF₂O)_{b}-(CF₂-(CF₂)_{z'}-CF₂O)_{c}

(where each of a, b, and c is an integer of 100 or less, z' is an integer of 1 or 2, a≥0, b≥0, c≥0, and a+b>0; particularly preferably, each of a, b, and c is an integer of 50 or less, a>0, b>0, and b/a is within the range of 0.1 to 10);

(2) -(C₃F₆O)_{c'}-(C₂F₄O)_{b}-(CFXO)ₜ-

(where Xs are independently selected from -F and CF₃, each of b, c', and t is an integer of 100 or less, c'>0, b≥0, and t≥0; particularly preferably, b>0, t>0, c'/b is within the range of 0.2 to 5.0, and (c'+b)/t is within the range of 5 to 50); or

(3) -(C₃F₆₀)_{c'}-(CFXO)ₜ-

(where Xs are independently selected from -F and CF₃, each of c' and t is an integer of 100 or less, c'>0, and t≥0; particularly preferably, t>0 and c'/t is within the range of 5 to 50).

Specific examples of the perfluoropolyether include products under the trade names GALDEN (registered trademark) "HT135" and GALDEN (registered trademark) "HT110" (both manufactured by Solvay), and the like.

### (1-6-5) Heat Transfer Fluid Compound That Can Be Additionally Contained

The heat transfer fluid according to the present disclosure may contain an additional heat transfer fluid compound (also called "an additional component") that is different from the above-mentioned three components (at least one selected from the group consisting of the compound represented by C₉F₁₈, perfluoroalkene ether, and perfluoropolyether, which are also collectively referred to as "the three components according to the present disclosure"). The additional component may be of one or more types, and the additional component may be a hexafluoropropene dimer, a hexafluoropropene tetramer, perfluorotripropylamine, perfluorotributylamine, and the like, for example.

The hexafluoropropene dimer may include (E)-1,1,1,2,3,4,5,5,5-nonafluoro-4-(trifluoromethyl)-2-pentene, (Z)-1,1,1,2,3,4,5,5,5-nonafluoro-4-(trifluoromethyl)-2-pentene, 1,1,3,4,4,5,5-nonafluoro-2-(trifluoromethyl)-2-pentene.

The hexafluoropropene tetramer may include 1,1,1,2,5,6,6,6-octafluoro-2,3,5-tris(trifluoromethyl)-4-(perfluoropropyl-2-yl)-3-hexene.

The perfluorotripropylamine is also referred to as tris(heptafluoropropyl)amine or N,N-bis(heptafluoropropyl)(heptafluoropropyl)amine, and represented by the general formula N(CF₂CF₂CF₃)ₙ(CF(CF₃)CF₃)₃₋ₙ (where n is an integer of 0 to 3). The perfluorotripropylamine may include only one type of compounds represented by the above general formula, or may include a plurality of types of them. N(CF₂CF₂CF₃)₃ is preferable, and N(CF₂CF₂CF₃)ₙ(CF(CF₃)CF₃)₃₋ₙ (where n is an integer of 0 to 2) may be included as impurities. Specific examples include a product under the trade name "Fluorinert (registered trademark)" (manufactured by 3M) (FC-3283) and the like.

The composition for heat transfer fluid according to the present disclosure, when containing both the three components according to the present disclosure and an additional component, preferably contains the three components according to the present disclosure in 80 mass% or more relative to the entire heat transfer fluid, more preferably 85 mass% or more, further preferably 90 mass% or more, most preferably 95 mass% or more. In the production method according to the present disclosure, the properties of the composition containing both the heat transfer fluid compound and conductive substances (the pre-purification composition) such as the kinematic viscosity, coagulation point, and boiling point can affect the efficiency of removal of the conductive substances from the pre-purification composition in relation to a certain purification treatment (a treatment performed with the use of at least one selected from the group consisting of filtration filter, ion-exchange resin, metal-ion removal filter, metal-ion removing agent, distillation, fractionation, centrifugation, and electrostatic attraction), and therefore it is preferable that the heat transfer fluid compound consist essentially of the three components according to the present disclosure; however, in the case where an additional component is contained, it is preferable that the three components be contained in 80 mass% or more relative to the entire heat transfer fluid as mentioned above.

Whether or not an additional component is contained in addition to the three components according to the present disclosure, the composition containing both the heat transfer fluid compound and conductive substances (the pre-purification composition) may be selected from a wide range including, for example, synthesized products and commercially available products of the three components according to the present disclosure as described above, as well as such synthesized products and commercially available products that were contaminated with conductive substances after a certain period of use as a heat transfer fluid.

### (1-6-6) Conductive Substances

Examples of the conductive substances include at least one selected from the group consisting of metals, metal ions, metal oxides, metal nitrides, carbon, conductive polymers, and superconducting ceramic. In the case where the heat transfer fluid containing conductive substances is used for the purpose of transferring heat to or from a device, the conductive substances can enter into the device through a gap to cause a short circuit. Moreover, they can cause clogging of the piping through which the heat transfer fluid is circulated, during repeated use of the heat transfer fluid. Hence, for the purpose of enhancing the performance of the heat transfer fluid, it is important to reduce the content of conductive substances. The shape and the size of the conductive substances vary depending on the type of the conductive substances, and typically, the conductive substances are of about 0.001 to 10 µm.

The type of the metal of the above-mentioned metals, metal ions, metal oxides, and metal nitrides as conductive substances may be, for example, Al, Ba, Be, Bi, Ca, Co, Cr, Cu, Fe, Ga, K, Li, Mg, Mn, Na, Ni, Pb, Sr, V, Zn, and the like.

The metal as a conductive substance may be either in the form of an elementary substance or in the form of alloy. Typically, the metal as a conductive substance is contained in the form of fine particles.

The ions as conductive substances include all forms of ions possible for the type of the metal. The ions as conductive substances may be present in the dissolved state as ions with certain valence, or may be present within the substance as ions such as, for example, in the coordinated state.

The metal oxide as a conductive substance may be an oxide of one type of metal, or may be an oxide of a plurality of types of metals (namely, a composite oxide).

The metal nitride as a conductive substance may be a nitride of one type of metal, or may be a nitride of a plurality of types of metals (namely, a composite nitride).

The carbon as a conductive substance may be carbon black, for example.

The conductive polymer as a conductive substance may be polyacetylene and/or polythiophene, for example.

The content of conductive substances in the pre-purification composition is not limited, and, for example, when it is 150 ppm by mass or more (or even 500 ppm by mass or more), the content of conductive substances can be effectively reduced by the production method according to the present disclosure. The content of conductive substances can be measured as described in Examples with the use of an inductively coupled plasma mass spectrometry instrument (ICP-MS).

The pre-purification composition may contain insoluble matter including solid matter such as resin fragments of the container and dirt and dust from the air, both conductive and not conductive; for example, when the content of insoluble matter of 5 µm or more is 50 pieces/mL or more (or even 100 pieces/mL or more), by performing the purification treatment according to the present disclosure capable of reducing the content of conductive substances, it is also possible to effectively reduce the content of insoluble matter. The number of insoluble matter (fine particles) of conductive substances contained in the composition can be measured as described in Examples with the use of a liquid particle counter.

### (1-6-7) Physical Properties of Pre-Purification Composition

The pre-purification composition is a composition containing both a heat transfer fluid and conductive substances, and in the production method according to the present disclosure, it is subjected to purification treatment performed with the use of at least one selected from the group consisting of filtration filter, ion-exchange resin, metal-ion removal filter, metal-ion removing agent, distillation, fractionation, centrifugation, and electrostatic attraction.

In view of performing the above-mentioned purification treatment to reduce the content of conductive substances, from the viewpoint of purification efficiency, it is preferable that the pre-purification composition have a low kinematic viscosity, a low coagulation point, and a high boiling point. Containing the above-mentioned three components according to the present disclosure as the main component (preferably in 80 mass% or more), the pre-purification composition tends to have such physical properties as a low kinematic viscosity, a low coagulation point, and a high boiling point.

The kinematic viscosity of the pre-purification composition at -40°C is preferably 15 cSt or less, more preferably 10 cSt or less, further preferably 7 cSt or less. When the kinematic viscosity is as low as 15 cSt or less, ease of filtration is enhanced, and thereby the efficiency of purification treatment with the use of a filtration filter, a metal-ion removal filter, and/or the like, in particular, can be enhanced. The method for measuring the kinematic viscosity at -40°C according to the present disclosure is described in Examples.

The coagulation point of the pre-purification composition is preferably -35°C or less, more preferably -50°C or less, further preferably -70°C or less, most preferably - 100°C or less. When the coagulation point is as low as -35°C or less, the purification treatment can be carried out at low temperatures and, therefore, loss of the heat transfer fluid due to evaporation and/or the like can be reduced and the efficiency of purification treatment can be enhanced. The method for measuring the coagulation point according to the present disclosure is described in Examples.

The boiling point of the pre-purification composition is 90°C or more, more preferably 95°C or more, further preferably 105°C or more. When the boiling point is as high as 90°C or more, for the same reason as mentioned above with respect to the low coagulation point, loss of the heat transfer fluid due to evaporation and/or the like can be reduced and the efficiency of purification treatment can be enhanced. The method for measuring the boiling point according to the present disclosure is described in Examples.

### (1-6-8) Purification Treatment of Pre-Purification Composition

In the production method according to the present disclosure, the pre-purification composition is subjected to purification treatment performed with the use of at least one selected from the group consisting of filtration filter, ion-exchange resin, metal-ion removal filter, metal-ion removing agent, distillation, fractionation, centrifugation, and electrostatic attraction.

These purification means can be used or carried out according to the conventional procedure, and in the production method according to the present disclosure, purification treatment with a filtration filter is particularly preferable. When a filtration filter is used, the upper limit and the lower limit to the filter pore size are not limited, and the upper limit can be set at 5 µm or less, or 1 µm or less, or 0.5 µm or less, or 0.1 µm or less, for example. Moreover, the lower limit can be set at 1.0 nm or more, or 0.5 nm or more, or 0.2 nm or more, or 0.1 nm or more, for example.

The ion-exchange resin may be either a cation-exchange resin or an anion-exchange resin. The anion-exchange resin can be an ion-exchange resin having amino groups and/or quaternary ammonium groups as functional groups, for example. Preferably, the ion-exchange resin is a strongly basic anion-exchange resin. The basicity of the anion-exchange resin can be adjusted by selecting the type of the polymer backbone and/or the functional group. The anion-exchange resin may be a commercially available product, and, for example, "Diaion (registered trademark) SA" products and the like manufactured by Mitsubishi Chemical, "A200" and the like manufactured by Purolite, and "Amberlite (registered trademark)" products and the like manufactured by Organo can be used. The cation-exchange resin can be an ion-exchange resin having carboxyl groups and/or sulfonic groups as functional groups, for example. The acidity of the cation-exchange resin can be adjusted by selecting the type of the polymer backbone and/or the functional group. The cation-exchange resin may be a commercially available product, and, for example, "Diaion (registered trademark) SK" products and the like manufactured by Mitsubishi Chemical, "C100" and the like manufactured by Purolite, and "Amberlite (registered trademark)" products and the like manufactured by Organo can be used.

The metal-ion removing agent may be a chelating agent, activated carbon, and/or the like. For example, the chelating agent can be CRB03, CRB05, and CR20 (all manufactured by Mitsubishi Chemical), Si-Thiol, Si-Thiourea, Si-TMT, Si-DMT, Si-SCX-2, Si-Amine, Si-Trisamine, Si-Imidazole, Si-TBD, and Si-PHI (all manufactured by SiliCycle), Muromac XMS-5418 (manufactured by Muromachi Chemicals), IRC76-HG, IRC748, and IRC747UPS (all manufactured by Organo), S910 (manufactured by Purolite), MPA (manufactured by Reaxa QuadraPure), and the like. For example, the activated carbon may be "SHIRASAGI (registered trademark)" and the like manufactured by Osaka Gas Chemicals, "Filtrasorb (registered trademark) CAL", "Diahope (registered trademark)", "Diasorb (registered trademark)", and the like manufactured by Calgon Carbon Japan, "Evadia (registered trademark)" products and the like manufactured by Swing, and the like.

By carrying out the above-mentioned purification treatment, it is possible to obtain a composition for heat transfer fluid (a post-purification composition) in which the content of conductive substances is reduced.

The above-described production method has the above-described purification treatment step, and it may also have other steps such as a step to mix an additive such as a stabilizer to the composition for heat transfer fluid.

### (2. Heat Transfer Fluid)

The composition for heat transfer fluid according to the present invention can be used together with a component other than the composition for heat transfer fluid (hereinafter, a fluid containing the composition for heat transfer fluid and a component other than the composition for heat transfer fluid is referred to as a heat transfer fluid). As long as the effect and the purpose of the heat transfer fluid according to the present disclosure are not impaired, the heat transfer fluid according to the present disclosure may contain a freely-selected additive other than the composition for heat transfer fluid in addition to the above-mentioned compounds represented by the formulae (I) to (III) as well as CₘF₂ₘ and/or CₙF₍₂ₙ₋₂₎, water, and/or fluoride ions. Examples of the freely-selected additive include a stabilizer and the like.

The stabilizer exhibits stabilizing effects and thereby it functions as a so-called acid scavenger or antioxidant. Examples of major stabilizing effects include effects to capture radicals produced in the system to prevent degradation of the HFP trimer, acid scavenging effects to capture acids produced in the system to prevent further degradation of the HFP trimer due to acid, and the like.

The stabilizer may be selected from a wide range of known stabilizers. Among these, preferably, for effectively reducing metal corrosion caused by the composition, one or more types of stabilizers selected from the group consisting of unsaturated alcohol-based stabilizers, nitro-based stabilizers, amine-based stabilizers, phenol-based stabilizers, and epoxy-based stabilizers are used.

The unsaturated alcohol-based stabilizer may be selected from a wide range of known ones. For example, one or more selected from the group consisting of 3-buten-2-ol, 2-buten-1-ol, 4-propen-1-ol, 1-propen-3-ol, 2-methyl-3-buten-2-ol, 3-methyl-3-buten-2-ol, 3-methyl-2-buten-1-ol, 2-hexen-1-ol, 2,4-hexadien-1-ol, and oleyl alcohol can be used.

The nitro-based stabilizer may be selected from a wide range of known ones. As aliphatic nitro compounds, nitromethane, nitroethane, 1-nitropropane, 2-nitropropane, and the like may be mentioned, for example. As aromatic nitro compounds, one or more selected from the group consisting of nitrobenzene, o-, m-, and p-dinitrobenzenes, o-, m-, and p-nitrotoluenes, dimethylnitrobenzene, m-nitroacetophenone, o-, m-, and p-nitrophenols, o-nitroanisole, m-nitroanisole, and p-nitroanisole can be used, for example.

The amine-based stabilizer may be selected from a wide range of known ones. For example, one or more selected from the group consisting of pentylamine, hexylamine, diisopropylamine, diisobutylamine, di-n-propylamine, diallylamine, triethylamine, N-methylaniline, pyridine, morpholine, N-methylmorpholine, triallylamine, allylamine, α-methylbenzylamine, dimethylamine, trimethylamine, ethylamine, diethylamine, triethylamine, propylamine, isopropylamine, dipropylamine, tripropylamine, butylamine, isobutylamine, dibutylamine, tributylamine, dipentylamine, tripentylamine, 2-ethylhexylamine, aniline, N,N-dimethylaniline, N,N-diethylaniline, ethylenediamine, propylenediamine, diethylenetriamine, tetraethylenepentamine, benzylamine, dibenzylamine, diphenylamine, and diethylhydroxylamine can be used.

The phenol-based stabilizer may be selected from a wide range of known ones. For example, one or more selected from the group consisting of 2,6-di-tert-butyl-4-methylphenol, 3-cresol, phenol, 1,2-benzenediol, 2-isopropyl-5-methylphenol, and 2-methoxyphenol can be used.

The epoxy-based stabilizer may be selected from a wide range of known ones. For example, one or more selected from the group consisting of butylene oxide, 1,2-propylene oxide, 1,2-butylene oxide, butyl glycidyl ether, diethylene glycol diglycidyl ether, and 1,2-epoxy-3-phenoxypropane can be used.

Use of a combination of stabilizers with different stabilizing effects can more effectively prevent degradation of the HFP trimer that can occur from various causes, and therefore, it is preferable to use one or more selected from the group consisting of the epoxy-based stabilizers, the unsaturated alcohol-based stabilizers, the nitro-based stabilizers, and the phenol-based stabilizers mentioned above.

From the viewpoint of effectively reducing acid release from the HFP trimer to reduce metal corrosion caused by the liquid composition, the content of the stabilizer in the entire heat transfer fluid is preferably 0.0001 mass% or more, more preferably 0.01 mass% or more. On the other hand, in consideration of avoiding undesired changes of the physical properties of the liquid composition due to excessive addition of stabilizers, the content of the stabilizer in the entire heat transfer fluid is preferably 10 mass% or less, more preferably 5 mass% or less.

### (3. Use of Composition for Heat Transfer Fluid or Heat Transfer Fluid)

The composition for heat transfer fluid or the heat transfer fluid according to the present disclosure is used for drawing heat away from various heat transfer targets or supplying heat to heat transfer targets. Examples of the heat transfer target according to the present disclosure include articles, apparatuses, and atmospheres that are to be cooled to, heated to, or maintained at an intended temperature. Examples of the heat transfer target include electric components, machine components, and optical components, as well as those after processing and those after assembly. Specific, non-limiting examples of the heat transfer target according to the present disclosure include wafers used for producing semiconductor devices, microprocessors, power control semiconductors, electric branch switches, power transformers, circuit boards, multichip modules, mounted and non-mounted semiconductor devices, chemical reactors, nuclear reactors, fuel cells, lasers, missile components, and the like.

The composition for heat transfer fluid or the heat transfer fluid according to the present disclosure is usable for two-phase immersion cooling and as chiller fluid and Rankine cycle working fluid.

Within a device designed to transfer heat by using the composition for heat transfer fluid or the heat transfer fluid according to the present disclosure, the composition for heat transfer fluid or the heat transfer fluid according to the present disclosure can be used to replace the heat transfer fluid in use in the device.

The composition for heat transfer fluid or the heat transfer fluid according to the present disclosure can be a drop-in replacement, a nearly drop-in replacement, or a retrofit replacement for the heat transfer fluid in use. "A drop-in replacement" means a replacement without requiring any changes to the device. "A nearly drop-in replacement" means a replacement requiring only few changes to the device. "A retrofit replacement" means a replacement requiring minimal changes (namely, not requiring extensive changes) to the device. Preferably, the composition for heat transfer fluid or the heat transfer fluid according to the present disclosure can be a drop-in replacement or a nearly drop-in replacement for the above-mentioned heat transfer fluid.

Determination that use as a drop-in replacement, a nearly drop-in replacement, or a retrofit replacement is possible can be made when all the following conditions are satisfied.
(i) The boiling point of the heat transfer fluid is at least about 80% or more, preferably at least about 85% or more, of the boiling point of the predecessor heat transfer fluid.
(ii) The pour point of the heat transfer fluid is equal to or less than the pour point of the predecessor heat transfer fluid.
(iii) The kinematic viscosity of the heat transfer fluid is at least about 200% or less, preferably at least about 150% or less, of the kinematic viscosity of the predecessor heat transfer fluid.
(iv) The heat transfer fluid is compatible with the predecessor heat transfer fluid at a freely-selected proportion.

When the boiling point of the composition for heat transfer fluid or the heat transfer fluid according to the present disclosure is designed to be at least about 80% or more, preferably at least about 85% or more, of the boiling point of the predecessor heat transfer fluid, occurrence of cavitation and leakage from the apparatus can be reduced. The upper limit to the boiling point of the heat transfer fluid is not limited, and, for example, it may be at least about 130% or less of the boiling point of the predecessor heat transfer fluid.

When the pour point of the composition for heat transfer fluid or the heat transfer fluid according to the present disclosure is designed to be equal to or less than the pour point of the predecessor heat transfer fluid, use at a temperature less than the conventional operational temperature is possible and, thereby, the operational temperature range can be extended. The upper limit to the pour point of the heat transfer fluid is not limited, and, for example, it may be equal to or less than the temperature 30°C more than the pour point of the predecessor heat transfer fluid.

When the kinematic viscosity of the composition for heat transfer fluid or the heat transfer fluid according to the present disclosure is designed to be at least about 200% or less, preferably at least about 150% or less, of the kinematic viscosity of the predecessor heat transfer fluid, an increase of power consumption can be reduced or power consumption can be reduced. Comparison of the kinematic viscosity is preferably performed in terms of the kinematic viscosity at the operational temperature, but this is not limitative, and the comparison can be performed in terms of the kinematic viscosity at a temperature from -20°C to -40°C, for example, and more specifically, it can be performed at -20°C.

When the composition for heat transfer fluid or the heat transfer fluid according to the present disclosure is compatible with the predecessor heat transfer fluid at a freely-selected proportion, replacing operation is easier to carry out.

Furthermore, when the following conditions are satisfied, the composition for heat transfer fluid or the heat transfer fluid according to the present disclosure is more suitable as a drop-in replacement, a nearly drop-in replacement, or a retrofit replacement.

(v) The composition for heat transfer fluid or the heat transfer fluid according to the present disclosure has a dielectric constant that is 120% or less of that of the predecessor heat transfer fluid.

(vi) The composition for heat transfer fluid or the heat transfer fluid according to the present disclosure has a dielectric strength that is 90% or more of that of the predecessor heat transfer fluid.

(vii) The composition for heat transfer fluid or the heat transfer fluid according to the present disclosure has a specific heat that is 90% or more of that of the predecessor heat transfer fluid.

(viii) The composition for heat transfer fluid or the heat transfer fluid according to the present disclosure has a thermal conductivity that is 90% or more of that of the predecessor heat transfer fluid.

When having a dielectric constant designed to be 120% or less of the dielectric constant of the predecessor heat transfer fluid, the composition for heat transfer fluid or the heat transfer fluid according to the present disclosure can be suitably used as a replacement composition. The lower limit to the dielectric constant of the heat transfer fluid is not limited, and, for example, it may be 80% or more of the dielectric constant of the predecessor heat transfer fluid.

When having a dielectric strength designed to be 90% or more of the dielectric strength of the predecessor heat transfer fluid, the composition for heat transfer fluid or the heat transfer fluid according to the present disclosure can be suitably used as a replacement composition. The upper limit to the dielectric strength of the heat transfer fluid is not limited, and, for example, it may be 120% or less of the dielectric strength of the predecessor heat transfer fluid.

When having a specific heat designed to be 90% or more of the specific heat of the predecessor heat transfer fluid, the composition for heat transfer fluid or the heat transfer fluid according to the present disclosure can be suitably used as a replacement composition. The upper limit to the specific heat of the heat transfer fluid is not limited, and, for example, it may be 120% or less of the specific heat of the predecessor heat transfer fluid.

When having a thermal conductivity designed to be 90% or more of the thermal conductivity of the predecessor heat transfer fluid, the composition for heat transfer fluid or the heat transfer fluid according to the present disclosure can be suitably used as a replacement composition. The upper limit to the thermal conductivity of the heat transfer fluid is not limited, and, for example, it may be 120% or less of the thermal conductivity of the predecessor heat transfer fluid.

The boiling point of the composition for heat transfer fluid or the heat transfer fluid according to the present disclosure may be preferably 105°C or more, more preferably 108°C or more. The upper limit to the boiling point of the composition for heat transfer fluid or the heat transfer fluid according to the present disclosure is not limited, and, for example, it may be 150°C or less, or 130°C or less, or 120°C or less.

The pour point of the composition for heat transfer fluid or the heat transfer fluid according to the present disclosure may be preferably -80°C or less, more preferably -100°C or less, further preferably -110°C or less. The lower limit to the pour point of the composition for heat transfer fluid or the heat transfer fluid according to the present disclosure is not limited, and, for example, it may be -180°C or more, or - 160°C or more.

The kinematic viscosity of the composition for heat transfer fluid or the heat transfer fluid according to the present disclosure at -20°C may be preferably 6.0 cSt or less, more preferably 5.0 cSt or less, further preferably 4.0 cSt or less, further more preferably 3.5 cSt or less. The lower limit to the kinematic viscosity of the composition for heat transfer fluid or the heat transfer fluid according to the present disclosure is not limited, and, for example, it may be 1.0 cSt or more.

The dielectric constant of the composition for heat transfer fluid or the heat transfer fluid according to the present disclosure may be preferably 3.0 or less, more preferably 2.5 or less, further preferably 2.0 or less. The lower limit to the dielectric constant of the composition for heat transfer fluid or the heat transfer fluid according to the present disclosure is not limited, and, for example, it may be 1.1 or more.

The dielectric strength of the composition for heat transfer fluid or the heat transfer fluid according to the present disclosure may be preferably 40 kV or more, more preferably 50 kV or more. The upper limit to the dielectric strength of the composition for heat transfer fluid or the heat transfer fluid according to the present disclosure is not limited, and, for example, it may be 150 kV or less, or 100 kV or less.

The specific heat of the composition for heat transfer fluid or the heat transfer fluid according to the present disclosure at 30°C may be preferably 800 J/kg·K or more, more preferably 900 J/kg·K or more, further preferably 1000 J/kg·K or more. The upper limit to the specific heat of the composition for heat transfer fluid or the heat transfer fluid according to the present disclosure is not limited, and, for example, it may be 2000 J/kg·K or less, or 1500 J/kg·K or less.

The thermal conductivity of the composition for heat transfer fluid or the heat transfer fluid according to the present disclosure at 30°C may be preferably 0.055 W/mK or more, more preferably 0.060 W/mK or more, further preferably 0.065 W/mK or more. The upper limit to the thermal conductivity of the composition for heat transfer fluid or the heat transfer fluid according to the present disclosure is not limited, and, for example, it may be 0.090 W/mK or less, or 0.080 W/mK or less.

The boiling point of the composition for heat transfer fluid or the heat transfer fluid according to the present disclosure is measured by DSC (differential scanning calorimetry), as a temperature at which an endothermic peak is observed while the temperature is being raised from 25°C at 5°C/minute.

The pour point of the composition for heat transfer fluid or the heat transfer fluid according to the present disclosure is measured by DSC, as a temperature at which an endothermic peak is observed while the temperature is being raised at 5°C/minute after lowered with liquid nitrogen to the coagulation point or less.

The dielectric constant of the composition for heat transfer fluid or the heat transfer fluid according to the present disclosure is a value at a frequency of 1 kHz measured by a capacitance method in an environment at a temperature of 25°C and a humidity of 60%.

Each of the kinematic viscosity and the density of the composition for heat transfer fluid or the heat transfer fluid according to the present disclosure is a value measured with a kinematic viscometer, SVM3001, manufactured by Anton Paar.

The dielectric strength of the composition for heat transfer fluid or the heat transfer fluid according to the present disclosure is the breakdown voltage measured at the time when a liquid specimen is made to enter into the gaps between spherical electrodes aligned at regular intervals and then the voltage is raised at a steady rate. Measurement conditions are as described below.
Electrode shape: Spherical (φ12.5 mm)
Intervals between electrodes: 2.5 mm
Pressure-raising rate: 2 kV/second
Atmosphere of measurement: Air (22°C, 57%RH)

The specific heat of the composition for heat transfer fluid or the heat transfer fluid according to the present disclosure is a value obtained by DSC under conditions described below.
Measurement apparatus: Differential scanning calorimeter DSC8500 manufactured by Perkin-Elmer
Temperature raising rate: 10°C/minute
Reference specimen: Sapphire (-Al2O3)
Atmosphere: In dry nitrogen stream
Specimen container: Hermetically sealed aluminum container

The thermal conductivity of the composition for heat transfer fluid or the heat transfer fluid according to the present disclosure is a value obtained by a transient hot-wire method.

The compatibility of the composition for heat transfer fluid or the heat transfer fluid according to the present disclosure is determined by checking if it is compatible with a target solvent when mixed. Herein, "being compatible" means that the two are mixed uniformly, in other words, phase separation does not occur.

### (4. Heat Transfer Apparatus)

The present disclosure further discloses a heat transfer apparatus comprising a device and a mechanism for transferring heat to the device or from the device, wherein the mechanism comprises the above-mentioned composition for heat transfer fluid.

Examples of the device include computers, server computers, servers including blade servers; disk array/storage systems; storage area networks; network-attached storages; storage communications systems; workstations; routers; telecommunications infrastructures/switches; wired, optical, and wireless communications apparatuses; cell processing apparatuses; printers; power source apparatuses; displays; optical apparatuses; measurement systems including hand-held systems; military electronic devices; and the like.

Semiconductor devices are heat-generating devices to be mounted on the device, such as CPUs, GPUs, SSDs, and the like, for example, and a semiconductor device is made of a single element such as silicon and germanium, a compound semiconductor such as gallium arsenide (GaAs), gallium phosphide (GaP), indium phosphide (InP), gallium nitride (GaN), and silicon carbide (SiC), and/or the like, for example.

When the device is a server computer, one logic board or a plurality of logic boards are accommodated in the internal space. The logic board comprises many heat-generating electronic components including at least one processor such as CPU and/or GPU. In addition to them, other heat-generating components for computers such as those listed below, for example, can be used: chipsets; memories, graphics chips, network chips, RAMs, power source apparatuses, daughter cards; and storage drives such as solid state drives and mechanical hard drives.

The heat transfer apparatus is a heat transfer apparatus that transfers heat to and from a heat transfer target by using the above-mentioned heat transfer fluid, and the supplying and receiving heat (heat transfer) is performed through thermal contact with the heat transfer target. For example, drawing heat away from the heat transfer target is cooling, and supplying heat is heating. Different mechanisms may be used for cooling and heating, respectively, or a singe heat transfer apparatus may be used for both cooling and heating.

The heat transfer apparatus is not limited and, for example, it may be a pump, a valve, a fluid containment system, a pressure control system, a cooler, a heat exchanger, a heat source, a heat sink, a refrigeration system, an active temperature control system, a passive temperature control system, and/or the like.

More specific examples include a temperature-controlled wafer chuck installed inside a plasma enhanced chemical vapor deposition (PECVD) tool, a temperature-controlled test head for die performance testing, a temperature-controlled workspace inside a semiconductor process device, a reservoir for thermal shock test bath liquid, a thermostatic chamber, and the like.

Examples of the heat transfer target to be brought into thermal contact with the heat transfer apparatus include articles, apparatuses, and atmospheres that are cooled to, heated to, or maintained at an intended temperature. Examples of the heat transfer target include electric components, machine components, and optical components, as well as those after processing and those after assembly. Specific, non-limiting examples of the heat transfer target according to the present disclosure include microprocessors, wafers used for producing semiconductor devices, power control semiconductors, electric branch switches, power transformers, circuit boards, multichip modules, mounted and non-mounted semiconductor devices, chemical reactors, nuclear reactors, fuel cells, lasers, missile components, and the like.

### (5. Heat Transfer Method)

The present disclosure also discloses a heat transfer method comprising a step of preparing a device, and a step of transferring heat to the device or from the device with the use of the above-mentioned composition for heat transfer fluid. Here, the heat transfer apparatus is placed in thermal contact with the device so as to make heat transfer possible. When placed in thermal contact with the device, the heat transfer apparatus can draw heat away from the device, or supply heat to the device, or maintain the device at a selected temperature or within a selected temperature range. The direction of heat flow (from the device or to the device) depends on the relative temperature difference between the device and the heat transfer apparatus.

Thus far, embodiments of the present invention are described, but it is obvious that the present invention is by no means limited to those examples and can be implemented in various configurations without departing from the gist of the present invention.

### Examples

In the following, a more detailed description will be given of embodiments of the present invention by way of Examples, but these Examples are not intended to limit the scope of the present invention.

### (Production Example 1)

Based on a method described in Chem Bar (1973), vol. 106, pp 2950-2959, an HFP trimer was obtained. The HFP trimer thus obtained was purified by distillation for removal of impurities such as hexafluoropropene dimers and tetramers. The purified HFP trimer was distillated and thereby separated into compounds represented by the formulae (I), (II), and (III). Each HFP trimer thus separated was dehydrated on silica gel.

The compounds represented by the formulae (I), (II), and (III) thus obtained were mixed in such a manner that the ratio between the compounds represented by the formulae (I), (II), and (III) became as specified in the table below, and thereby trimer mixtures 1 to 4 were obtained.

**[Table 1]**

| Trimer mixture | Formula (I) | Formula (II) | Formula (III) |
|---|---|---|---|
| 1 | 1.5 mass% | 35.5 mass% | 63.0 mass% |
| 2 | 52.0 mass% | 16.0 mass% | 32.0 mass% |
| 3 | 77.0 mass% | 8.0 mass% | 15.0 mass% |
| 4 | 90.0 mass% | 5.0 mass% | 5.0 mass% |

### (Production Example 2)

Based on a method described in Journal of the Chemical Society, Perkin Transactions 1: Organic and Bio-Organic Chemistry (1981), vol. 4, pp 1064-1067, C₉F₁₆ was synthesized.

### (Production Example 3)

As C₈F₁₆, a reagent manufactured by Wako Pure Chemical Industries was purchased.

### (Examples 1 to 43)

The trimer mixtures 1 to 4 and an additive, which was C₉F₁₆ of Production Example 2 or C₈F₁₆ of Production Example 3, were mixed in the proportion specified in the table below, and thereby compositions for heat conduction fluid of Examples 1 to 43 were obtained.

### (Stability Test)

The composition for heat conduction fluid thus obtained was placed and hermetically sealed in an autoclave made of SUS, and then heated and maintained under the conditions specified in the table below.

### (Measurement of Concentration of Fluoride Ions)

To a specimen, an equal amount (weight) of distilled water was added, and the resultant was shaken for about 20 seconds to extract F ions into the aqueous layer. Then, 2.5 to 3.0 mL of the aqueous layer was taken out with a pipette and mixed with twice the amount of TISAB solution (total ionic strength adjustment buffer, manufactured by HORIBA), and the resulting liquid was used as a sample for measurement with a fluoride ion meter (manufactured by HORIBA). In consideration of the dilution with the TISAB solution, three times the measured value is the concentration of fluoride ions. Results are given in the table below.

**[Table 2]**

| | Trimer mixture No. | Trimer content (g) | Additive | Additive content (mg) | wt% |
|---|---|---|---|---|---|
| Ex. 1 | 1 | 10 | C₉F₁₆ | 140 | 1.39 |
| Ex. 2 | 2 | 10 | C₉F₁₆ | 0.01 | 0.0001 |
| Ex. 3 | 2 | 10 | C₉F₁₆ | 1 | 0.01 |
| Ex. 4 | 2 | 10 | C₉F₁₆ | 10 | 0.1 |
| Ex. 5 | 2 | 10 | C₉F₁₆ | 0.01 | 0.0001 |
| Ex. 6 | 2 | 10 | C₉F₁₆ | 1 | 0.01 |
| Ex. 7 | 2 | 10 | C₉F₁₆ | 10 | 0.1 |
| Ex. 8 | 3 | 10 | C₉F₁₆ | 0.01 | 0.0001 |
| Ex. 9 | 3 | 10 | C₉F₁₆ | 1 | 0.01 |
| Ex. 10 | 3 | 10 | C₉F₁₆ | 10 | 0.1 |
| Ex. 11 | 3 | 10 | C₉F₁₆ | 0.01 | 0.0001 |
| Ex. 12 | 3 | 10 | C₉F₁₆ | 1 | 0.01 |
| Ex. 13 | 3 | 10 | C₉F₁₆ | 10 | 0.1 |
| Ex. 14 | 4 | 10 | C₉F₁₆ | 0.01 | 0.0001 |
| Ex. 15 | 4 | 10 | C₉F₁₆ | 1 | 0.01 |
| Ex. 16 | 4 | 10 | C₉F₁₆ | 10 | 0.1 |
| Ex. 17 | 4 | 10 | C₉F₁₆ | 0.01 | 0.0001 |
| Ex. 18 | 4 | 10 | C₉F₁₆ | 1 | 0.01 |
| Ex. 19 | 4 | 10 | C₉F₁₆ | 10 | 0.1 |
| Ex. 20 | 2 | 10 | C₈F₁₆ | 0.01 | 0.0001 |
| Ex. 21 | 2 | 10 | C₈F₁₆ | 1 | 0.01 |
| Ex. 22 | 2 | 10 | C₈F₁₆ | 10 | 0.1 |
| Ex. 23 | 2 | 10 | C₈F₁₆ | 100 | 1.0 |
| Ex. 24 | 2 | 10 | C₈F₁₆ | 0.01 | 0.0001 |
| Ex. 25 | 2 | 10 | C₈F₁₆ | 1 | 0.01 |
| Ex. 26 | 2 | 10 | C₈F₁₆ | 10 | 0.1 |
| Ex. 27 | 2 | 10 | C₈F₁₆ | 100 | 1.0 |
| Ex. 28 | 3 | 10 | C₈F₁₆ | 0.01 | 0.0001 |
| Ex. 29 | 3 | 10 | C₈F₁₆ | 1 | 0.01 |
| Ex. 30 | 3 | 10 | C₈F₁₆ | 10 | 0.1 |
| Ex. 31 | 3 | 10 | C₈F₁₆ | 100 | 1.0 |
| Ex. 32 | 3 | 10 | C₈F₁₆ | 0.01 | 0.0001 |
| Ex. 33 | 3 | 10 | C₈F₁₆ | 1 | 0.01 |
| Ex. 34 | 3 | 10 | C₈F₁₆ | 10 | 0.1 |
| Ex. 35 | 3 | 10 | C₈F₁₆ | 100 | 1.0 |
| Ex. 36 | 4 | 10 | C₈F₁₆ | 0.01 | 0.0001 |
| Ex. 37 | 4 | 10 | C₈F₁₆ | 1 | 0.01 |
| Ex. 38 | 4 | 10 | C₈F₁₆ | 10 | 0.1 |
| Ex. 39 | 4 | 10 | C₈F₁₆ | 100 | 1.0 |
| Ex. 40 | 4 | 10 | C₈F₁₆ | 0.01 | 0.0001 |
| Ex. 41 | 4 | 10 | C₈F₁₆ | 1 | 0.01 |
| Ex. 42 | 4 | 10 | C₈F₁₆ | 10 | 0.1 |
| Ex. 43 | 4 | 10 | C₈F₁₆ | 100 | 1.0 |
| Comp. Ex. 1 | 2 | 10 | - | - | - |
| Comp. Ex. 2 | 2 | 10 | - | - | - |

**[Table 3]**

| | Initial concentration of F ions (ppm by mass) | Stability test | | After-test concentration of F ions (ppm by mass) |
|---|---|---|---|---|
| | | Temperature (°C) | Time (hour) | |
| Ex. 1 | 0.6 | 100 | 168 | 1.2 |
| Ex. 2 | 0.6 | 100 | 168 | 3.3 |
| Ex. 3 | 0.6 | 100 | 168 | 2.1 |
| Ex. 4 | 0.6 | 100 | 168 | 1.8 |
| Ex. 5 | 0.6 | 200 | 168 | 3.6 |
| Ex. 6 | 0.6 | 200 | 168 | 2.7 |
| Ex. 7 | 0.6 | 200 | 168 | 2.1 |
| Ex. 8 | 0.6 | 100 | 168 | 3.6 |
| Ex. 9 | 0.6 | 100 | 168 | 2.4 |
| Ex. 10 | 0.6 | 100 | 168 | 1.8 |
| Ex. 11 | 0.6 | 200 | 168 | 4.2 |
| Ex. 12 | 0.6 | 200 | 168 | 3.6 |
| Ex. 13 | 0.6 | 200 | 168 | 2.7 |
| Ex. 14 | 0.6 | 100 | 168 | 3.9 |
| Ex. 15 | 0.6 | 100 | 168 | 2.7 |
| Ex. 16 | 0.6 | 100 | 168 | 2.1 |
| Ex. 17 | 0.6 | 200 | 168 | 4.5 |
| Ex. 18 | 0.6 | 200 | 168 | 3.9 |
| Ex. 19 | 0.6 | 200 | 168 | 3.3 |
| Ex. 20 | 0.6 | 100 | 168 | 1.2 |
| Ex. 21 | 0.6 | 100 | 168 | 0.9 |
| Ex. 22 | 0.6 | 100 | 168 | 0.3 |
| Ex. 23 | 0.6 | 100 | 168 | 0.3 |
| Ex. 24 | 0.6 | 200 | 168 | 1.5 |
| Ex. 25 | 0.6 | 200 | 168 | 1.2 |
| Ex. 26 | 0.6 | 200 | 168 | 0.9 |
| Ex. 27 | 0.6 | 200 | 168 | 0.3 |
| Ex. 28 | 0.6 | 100 | 168 | 1.5 |
| Ex. 29 | 0.6 | 100 | 168 | 1.2 |
| Ex. 30 | 0.6 | 100 | 168 | 0.9 |
| Ex. 31 | 0.6 | 100 | 168 | 0.6 |
| Ex. 32 | 0.6 | 200 | 168 | 1.8 |
| Ex. 33 | 0.6 | 200 | 168 | 1.5 |
| Ex. 34 | 0.6 | 200 | 168 | 1.2 |
| Ex. 35 | 0.6 | 200 | 168 | 0.6 |
| Ex. 36 | 0.6 | 100 | 168 | 2.1 |
| Ex. 37 | 0.6 | 100 | 168 | 1.5 |
| Ex. 38 | 0.6 | 100 | 168 | 0.9 |
| Ex. 39 | 0.6 | 100 | 168 | 0.6 |
| Ex. 40 | 0.6 | 200 | 168 | 1.8 |
| Ex. 41 | 0.6 | 200 | 168 | 1.8 |
| Ex. 42 | 0.6 | 200 | 168 | 1.2 |
| Ex. 43 | 0.6 | 200 | 168 | 0.9 |
| Comp. Ex. 1 | 0.6 | 100 | 168 | 45 |
| Comp. Ex. 2 | 0.6 | 200 | 168 | 99 |

From the above results, it is demonstrated that Examples 1 to 43 containing C₉F₁₆ or C₈F₁₆ did not show an increase of fluorine concentrations even after long-term storage at high temperatures and therefore they were stable.

### <Measurement of Boiling Point, Pour Point, and Dielectric Constant>

The boiling point of the trimer mixture was measured by DSC (differential scanning calorimetry), as a temperature at which an endothermic peak was observed while the temperature was being raised from 25°C at 5°C/min. The pour point was measured by DSC, as a temperature at which an endothermic peak was observed while the temperature was being raised at 5°C/min after lowered with liquid nitrogen to the coagulation point or less. The dielectric constant was a value at a frequency of 1 kHz measured by a capacitance method in an environment at a temperature of 25°C and a humidity of 60%.

### <Measurement of Kinematic Viscosity and Density>

The kinematic viscosity and the density of the trimer mixture were measured with a kinematic viscometer, SVM3001, manufactured by Anton Paar.

### <Measurement of Specific Heat>

The specific heat of the trimer mixture was measured by DSC. The conditions of the measurement were as described below.
Measurement apparatus: Differential scanning calorimeter DSC8500 manufactured by Perkin-Elmer
Temperature raising rate: 10°C/minute
Reference specimen: Sapphire (-Al2O3)
Atmosphere: In dry nitrogen stream
Specimen container: Hermetically sealed aluminum container

### <Measurement of Thermal Conductivity>

The thermal conductivity of the trimer mixture was measured by a transient hot-wire method.

### <Compatibility>

The compatibility of the trimer mixture was evaluated by using a mixture of equal amounts of the below-listed three types of solvents and each trimer mixture.
Galden HT135 (manufactured by Solvay)
SF-10 (manufactured by Chemours)
FC3283 (manufactured by 3M)

**[Table 4]**

| | Mixture 1 | Mixture 2 | Mixture 3 | Mixture 4 |
|---|---|---|---|---|
| Boiling point (°C) | 112 | 109 | 109 | 108.5 |
| Pour point (°C) | -110 | <-130 | <-130 | <-130 |
| Dielectric constant (1 kHz) | 1.97 | 2.00 | 2.00 | 2.00 |
| Kinematic viscosity (cSt) @-20°C | 4.8 | 2.6 | 2.5 | 2.4 |
| Dielectric strength (kV) | >40 | >40 | >40 | >40 |
| Specific heat (J/kg·K) @30°C | 1100 | 1060 | 1060 | 1060 |
| Thermal conductivity (W/mK) @30°C | 0.074 | 0.064 | 0.062 | 0.060 |
| Compatibility | Compatible with three types | Compatible with three types | Compatible with three types | Compatible with three types |

### (Examples 44 to 59)

The trimer mixtures 1 to 4 and water were mixed in the amounts specified in the table below, and thereby compositions of Examples 44 to 59 were obtained.

**[Table 5]**

| | Trimer mixture | Amount of added trimer (g) | Amount of added water (mg) |
|---|---|---|---|
| Ex. 44 | 1 | 26 | 1.82 |
| Ex. 45 | 2 | 10 | 0.01 |
| Ex. 46 | 2 | 10 | 1 |
| Ex. 47 | 2 | 10 | 10 |
| Ex. 48 | 2 | 10 | 0.01 |
| Ex. 49 | 2 | 10 | 1 |
| Ex. 50 | 2 | 10 | 10 |
| Ex. 51 | 3 | 10 | 0.01 |
| Ex. 52 | 3 | 10 | 1 |
| Ex. 53 | 3 | 10 | 10 |
| Ex. 54 | 3 | 10 | 0.01 |
| Ex. 55 | 3 | 10 | 1 |
| Ex. 56 | 3 | 10 | 10 |
| Ex. 57 | 4 | 10 | 0.01 |
| Ex. 58 | 4 | 10 | 1 |
| Ex. 59 | 4 | 10 | 10 |
| Comp. Ex. 3 | 2 | 10 | - |
| Comp. Ex. 4 | 2 | 10 | - |
| Comp. Ex. 5 | 2 | 10 | 150 |
| Comp. Ex. 6 | 2 | 10 | 150 |

### (Stability Test)

Each of the compositions of Examples 44 to 59 and Comparative Examples 3 to 6 was placed and hermetically sealed in an autoclave made of SUS, and then heated and maintained under the conditions specified in the table below.

### (Measurement of Dielectric Strength)

A liquid specimen was made to enter into the gaps between spherical electrodes aligned at regular intervals, followed by raising the voltage at a steady rate, and the breakdown voltage was measured and regarded as the dielectric strength. Specific conditions of the measurement were as described below.
Electrode shape: Spherical (φ12.5 mm)
Intervals between electrodes: 2.5 mm
Pressure-raising rate: 2 kV/second
Atmosphere of measurement: Air (22°C, 57%RH)

**[Table 6]**

| | Concentration of F ions (initial) (ppm by mass) | Stability test | | Concentration of F ions (after test) (ppm by mass) | Dielectric strength (kV) |
|---|---|---|---|---|---|
| | | Temperature (°C) | Time (hour) | | |
| Ex. 44 | 0.6 | 150 | 168 | 0.356 | 64 |
| Ex. 45 | 0.6 | 100 | 168 | 2.1 | 68 |
| Ex. 46 | 0.6 | 100 | 168 | 3.6 | 60 |
| Ex. 47 | 0.6 | 100 | 168 | 4.8 | 61 |
| Ex. 48 | 0.6 | 200 | 168 | 3.0 | 68 |
| Ex. 49 | 0.6 | 200 | 168 | 5.4 | 63 |
| Ex. 50 | 0.6 | 200 | 168 | 6.6 | 61 |
| Ex. 51 | 0.6 | 100 | 168 | 3.3 | 66 |
| Ex. 52 | 0.6 | 100 | 168 | 4.8 | 54 |
| Ex. 53 | 0.6 | 100 | 168 | 6.3 | 52 |
| Ex. 54 | 0.6 | 200 | 168 | 4.8 | 68 |
| Ex. 55 | 0.6 | 200 | 168 | 7.8 | 63 |
| Ex. 56 | 0.6 | 200 | 168 | 12.0 | 58 |
| Ex. 57 | 0.6 | 100 | 168 | 3.6 | 48 |
| Ex. 58 | 0.6 | 100 | 168 | 5.4 | 44 |
| Ex. 59 | 0.6 | 100 | 168 | 7.5 | 46 |
| Comp. Ex. 3 | 0.6 | 100 | 168 | 102 | 39 |
| Comp. Ex. 4 | 0.6 | 200 | 168 | 303 | 64 |
| Comp. Ex. 5 | 0.6 | 100 | 168 | 210 | 68 |
| Comp. Ex. 6 | 0.6 | 200 | 168 | 492 | 60 |

From the above results, it is demonstrated that Examples 44 to 59 containing certain amounts of water did not show an increase of fluorine concentrations even after long-term storage at high temperatures and therefore they were stable. Moreover, it is also demonstrated that Examples 44 to 59 had high dielectric strength.

### (Examples 60 to 83)

Anhydrous hydrofluoric acid was added to the trimer mixtures 1 to 4 to prepare base liquids, and the resultants were diluted with the corresponding trimer mixtures, respectively, to form compositions of Examples 60 to 83 having concentrations of fluoride ions as specified in the table below.

### (Stability Test)

A specimen was placed and hermetically sealed in an autoclave made of SUS, and then heated and maintained under the conditions specified in the table below.

### (Measurement of Purity of Hexafluoropropene Trimer)

The purity of the hexafluoropropene trimer before and after the stability test was measured by gas chromatography.

**[Table 7]**

| | Trimer mixture | Trimer content (g) | F ion amount (initial) (ppm by mass) |
|---|---|---|---|
| Ex. 60 | 1 | 26 | <0.001 |
| Ex. 61 | 1 | 9.2 | <0.001 |
| Ex. 62 | 1 | 8.8 | <0.001 |
| Ex. 63 | 2 | 10 | 0.6 |
| Ex. 64 | 2 | 10 | 0.6 |
| Ex. 65 | 2 | 10 | 0.6 |
| Ex. 66 | 2 | 10 | 0.6 |
| Ex. 67 | 2 | 10 | 0.6 |
| Ex. 68 | 2 | 10 | 0.6 |
| Ex. 69 | 2 | 10 | 0.6 |
| Ex. 70 | 3 | 10 | 0.6 |
| Ex. 71 | 3 | 10 | 0.6 |
| Ex. 72 | 3 | 10 | 0.6 |
| Ex. 73 | 3 | 10 | 0.6 |
| Ex. 74 | 3 | 10 | 0.6 |
| Ex. 75 | 3 | 10 | 0.6 |
| Ex. 76 | 3 | 10 | 0.6 |
| Ex. 77 | 4 | 10 | 0.6 |
| Ex. 78 | 4 | 10 | 0.6 |
| Ex. 79 | 4 | 10 | 0.6 |
| Ex. 80 | 4 | 10 | 0.6 |
| Ex. 81 | 4 | 10 | 0.6 |
| Ex. 82 | 4 | 10 | 0.6 |
| Ex. 83 | 4 | 10 | 0.6 |
| Comp. Ex. 7 | 2 | 10 | 0.6 |
| Comp. Ex. 8 | 2 | 10 | 0.6 |
| Comp. Ex. 9 | 2 | 10 | 0.6 |
| Comp. Ex. 10 | 2 | 10 | 0.6 |

**[Table 8]**

| | Stability test | | Initial purity (wt%) | After-test purity (wt%) |
|---|---|---|---|---|
| | Temperature (°C) | Time (hour) | | |
| Ex. 60 | 150 | 24 | 99.9 | 99.9 |
| Ex. 61 | 150 | 24 | 99.9 | 99.9 |
| Ex. 62 | 150 | 24 | 99.9 | 99.9 |
| Ex. 63 | 100 | 168 | 99.9 | 99.9 |
| Ex. 64 | 100 | 168 | 99.9 | 99.9 |
| Ex. 65 | 100 | 168 | 99.9 | 99.9 |
| Ex. 66 | 100 | 168 | 99.9 | 99.9 |
| Ex. 67 | 200 | 168 | 99.9 | 99.9 |
| Ex. 68 | 200 | 168 | 99.9 | 99.9 |
| Ex. 69 | 200 | 168 | 99.9 | 99.8 |
| Ex. 70 | 100 | 168 | 99.9 | 99.9 |
| Ex. 71 | 100 | 168 | 99.9 | 99.8 |
| Ex. 72 | 100 | 168 | 99.9 | 99.8 |
| Ex. 73 | 100 | 168 | 99.9 | 99.7 |
| Ex. 74 | 200 | 168 | 99.9 | 99.7 |
| Ex. 75 | 200 | 168 | 99.9 | 99.7 |
| Ex. 76 | 200 | 168 | 99.9 | 99.8 |
| Ex. 77 | 100 | 168 | 99.9 | 99.7 |
| Ex. 78 | 100 | 168 | 99.9 | 99.7 |
| Ex. 79 | 100 | 168 | 99.9 | 99.7 |
| Ex. 80 | 100 | 168 | 99.9 | 99.6 |
| Ex. 81 | 200 | 168 | 99.9 | 99.6 |
| Ex. 82 | 200 | 168 | 99.9 | 99.5 |
| Ex. 83 | 200 | 168 | 99.9 | 99.5 |
| Comp. Ex. 7 | 100 | 168 | 99.9 | 98.4 |
| Comp. Ex. 8 | 200 | 168 | 99.9 | 98.5 |
| Comp. Ex. 9 | 200 | 168 | 99.9 | 99.2 |
| Comp. Ex. 10 | 200 | 336 | 99.9 | 99.0 |

From the above results, it is demonstrated that in Examples 60 to 83 containing certain amounts of fluoride ions, the purity of the hexafluoropropene trimer remained high even after long-term storage at high temperatures.

The compounds represented by the formulae (I), (II), and (III) thus obtained were mixed in such a manner that the ratio between the compounds represented by the formulae (I), (II), and (III) became as specified in the table below, and thereby trimer mixtures 5 to 6 were obtained.

**[Table 9]**

| Trimer mixture | Formula (I) | Formula (II) | Formula (III) |
|---|---|---|---|
| 5 | 3.0 mass% | 33.0 mass% | 64.0 mass% |
| 6 | 99.5 mass% | 0.2 mass% | 0.3 mass% |

### (Production of Hexafluoropropene Tetramer)

### (Production Example 4)

Based on a method described in Tetrahedron Lett. 1974, 24, 2129-2132, a hexafluoropropene tetramer was obtained.

By mixing the trimer mixture 5 or 6 and the hexafluoropropene tetramer obtained in Production Example 4, it is possible to obtain the heat transfer fluid according to the present disclosure.

### <Evaluation Method>

### (Boiling Point)

The boiling point was measured by DSC, as a temperature at which an endothermic peak was observed while the temperature was being raised from 25°C at 5°C/minute.

### (Kinematic Viscosity)

The kinematic viscosity was a value at 25°C measured in accordance with JIS K 2283 with an Ubbelohde viscometer.

### (Dielectric Constant)

The dielectric constant was a value at a frequency of 1 kHz measured by a capacitance method in an environment at a temperature of 25°C and a humidity of 60%.

### (Examples 84 to 85)

The trimer mixture 5 and the hexafluoropropene tetramer were mixed in a weight ratio of 91:9 (Example 84), and the trimer mixture 6 and the hexafluoropropene tetramer were mixed in a weight ratio of 91:9 (Example 85). Physical properties were measured and results are given in Table 2.

**[Table 10]**

| | Boiling point (°C) | Dielectric constant | Kinematic viscosity (cSt) |
|---|---|---|---|
| Trimer mixture 5 | 115 | 2.0 | 1.4 |
| Trimer mixture 6 | 107 | 2.0 | 0.9 |

### <Examples 86 to 87>

In Examples 86 to 87, a pre-purification composition to be subjected to purification procedure (Composition 1 containing both a heat transfer fluid and conductive substances; 6FT) was prepared. Specifically, 750 g of DMF and 7.2 g of cesium fluoride were placed and hermetically sealed in an autoclave made of SUS. After air was evacuated from the autoclave, 2268 g of hexafluoropropylene was added over 4.5 hours while the temperature inside the autoclave was maintained at 70 to 110°C. From the reaction liquid thus obtained, the lower layer was separated and rinsed with ultrapure water, and thereby 2219 g of a composition containing an HFP trimer (Composition 1, a pre-purification composition) was obtained. GCFID and GC-MS analyses were carried out, and by an area normalization method, it was checked that the content of the HFP trimer was 87 mass% in the total amount of the composition regarded as 100 mass%, and in the total amount of the HFP trimer regarded as 100 mass%, the contents of the compounds represented by the formulae (I), (II), and (III) according to the present specification were 78 mass%, 9 mass%, and 13 mass%, respectively.

### <Example 88>

In the same manner as in Examples 86 to 87, the compounds represented by the formulae (I), (II), and (III) were isolated and mixed in such a manner that the contents of the compounds represented by the formulae (I), (II), and (III) became 55 mass%, 15 mass%, and 30 mass%, respectively, and thereby 2200 g of a composition was prepared.

### <Example 89>

In the same manner as in Examples 86 to 87, the compounds represented by the formulae (I), (II), and (III) were isolated and mixed in such a manner that the contents of the compounds represented by the formulae (I), (II), and (III) became 3 mass%, 33 mass%, and 64 mass%, respectively, and thereby 2200 g of a composition was prepared.

### <Examples 90>

In the same manner as in Examples 86 to 87, the compounds represented by the formulae (I), (II), and (III) were isolated and mixed in such a manner that the contents of the compounds represented by the formulae (I), (II), and (III) became 90 mass%, 5 mass%, and 5 mass%, respectively, and thereby 2200 g of a composition was prepared.

### (Measurement of Contents of Metals and Metal Ions in Pre-Purification Composition)

The content of conductive substances (specifically, in the present examples, the content of metals and metal ions) in the pre-purification composition was determined by the procedure described below with the use of an inductively coupled plasma mass spectrometry instrument (ICP-MS) (The same applies to the post-purification composition). The measured values are given in Table 1 below.
(1) The pre-purification composition in an amount of 2000 g was poured into a polytetrafluoroethylene (PTFE) beaker, and the beaker was placed on a hot plate at 250°C to let volatile components be volatilized.
(2) Nitric acid for trace amount analysis (manufactured by Wako Pure Chemical Industries) having a concentration of 70% was diluted with ultrapure water to form nitric acid having a concentration of about 4 mass%, and about 50 mL of the resultant was placed in the PTFE beaker.
(3) The PTFE beaker was placed on a hot plate at 150°C for 1 hour to let the metal remaining in the beaker dissolve.
(4) The amount of metal ions dissolved in nitric acid was measured on an ICP-MS. The amount of metal ions in nitric acid, the amount of nitric acid, and the amount of volatilized solution were used to calculate the content of metals and metal ions in the solution.
(5) All the procedures related to the above-mentioned measurement were carried out inside a Class 10000 clean room in which a KOACH table manufactured by Koken Ltd. was installed for ensuring a work environment equivalent to Class 1.

### (Measurement of Number of Insoluble Matter (Fine Particles) in Pre-Purification Composition)

The number of particles having particle sizes of 5.0 µm or more, the number of particles having particle sizes of not less than 1.0 µm and less than 5.0 µm, the number of particles having particle sizes of not less than 0.5 µm and less than 1.0 µm, and the number of particles having particle sizes of less than 0.3 µm and less than 0.5 µm in the pre-purification composition were measured with a liquid particle counter ("KL-22" manufactured by RION) at a temperature of 23°C (The same applies to the post-purification composition). All the procedures related to the above-mentioned measurement were carried out inside a Class 10000 clean room in which a KOACH table manufactured by Koken Ltd. was installed for ensuring a work environment equivalent to Class 1.

### (Measurement of Boiling Point, Coagulation Point, and Kinematic Viscosity of Pre-Purification Composition)

The boiling point was measured by DSC, as a temperature at which an endothermic peak was observed while the temperature was being raised from 25°C at 5°C/min. The coagulation point was measured by DSC, as a temperature at which an endothermic peak was observed while the temperature was being raised at 5°C/min after lowered with liquid nitrogen to -150°C or less (the temperature at which solidification was observed). The kinematic viscosity was measured in accordance with JIS K 2283 with an Ubbelohde viscometer. The measured values are given in Table 1 below.

### (Purification Treatment)

As the filtration apparatus, a multi-stage filtration apparatus was prepared that consisted of a required number of required units connected in series where each unit was formed of one filter contained in one vessel.

The pre-purification composition was placed in a pressurized vessel and cooled to -5°C or less, and then in a highly pure argon atmosphere, with the use of the above-mentioned filtration apparatus, pressure filtration was carried out to 0.02 MPa to form a post-purification composition. The filters used here in the units of the filtration apparatus were:
Filter A ("IonKleen SL" manufactured by Nihon Pall, with filtration area of 0.58 m2),
Filter B ("Ultipleat P-Nylon" manufactured by Nihon Pall, with pore size of 0.15 µm and filtration area of 1.2 m2), and
Filter C ("Ultipleat P-Nylon" manufactured by Nihon Pall, with pore size of 40 nm and filtration area of 1.2 m2)
and these were used as specified in Table 1. Results are given in Table 1.

From the results in Table 1, it is indicated that by subjecting the pre-purification composition to a certain purification treatment, it is possible to reduce the content of conductive substances (metals and metal ions, in particular).

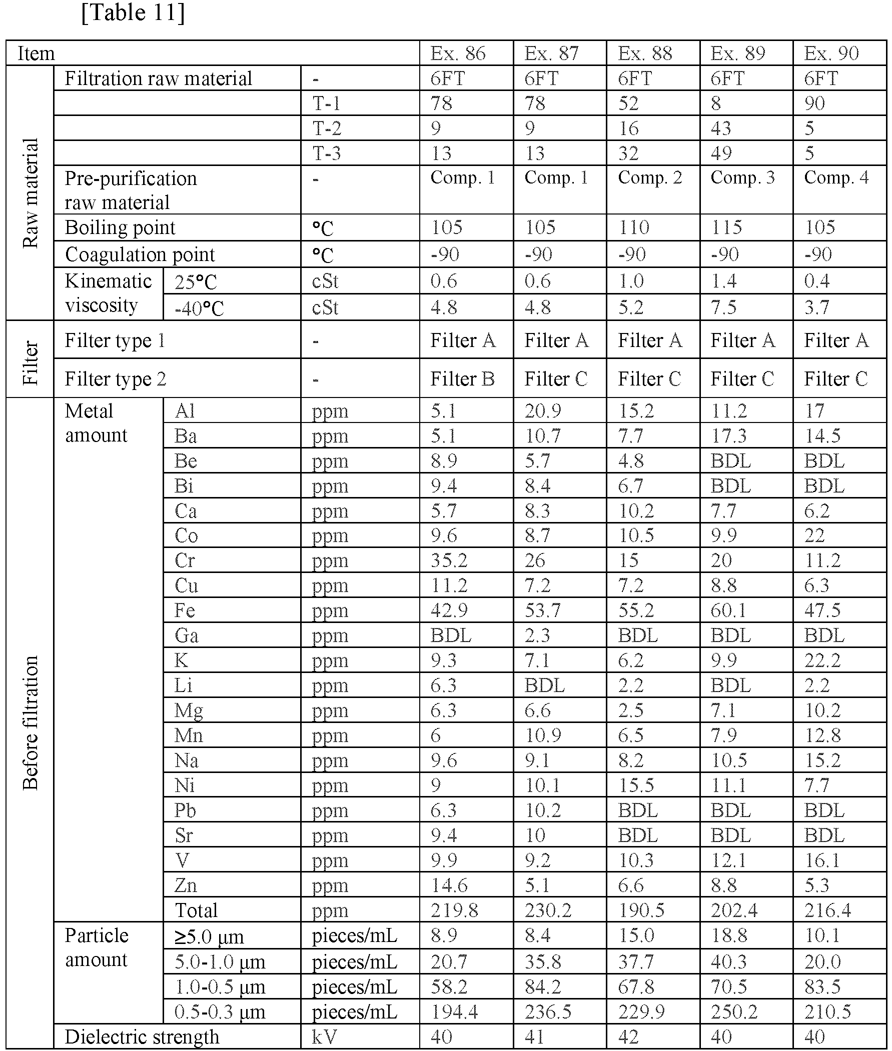

**[Table 12]**

| Item | | | | Ex. 86 | Ex. 87 | Ex. 88 | Ex. 89 | Ex. 90 |
|---|---|---|---|---|---|---|---|---|
| | Metal amount | Al | ppm | 3.61 | 5.6 | 6.6 | 2.3 | 5.8 |
| | | Ba | ppm | BDL | BDL | BDL | BDL | BDL |
| | | Be | ppm | BDL | BDL | BDL | BDL | BDL |
| | | Bi | ppm | 0.67 | BDL | BDL | BDL | BDL |
| | | Ca | ppm | 29.61 | 3.54 | 2.26 | 1.1 | 4 |
| | | Co | ppm | 0.01 | BDL | BDL | BDL | BDL |
| | | Cr | ppm | BDL | BDL | BDL | BDL | BDL |
| | | Cu | ppm | 4.66 | 0.37 | 0.5 | 1.1 | 0.5 |
| | | Fe | ppm | 1.65 | 0.1 | 0.1 | 0.5 | 0.1 |
| | | Ga | ppm | BDL | BDL | BDL | BDL | BDL |
| | | K | ppm | BDL | BDL | BDL | BDL | BDL |
| | | Li | ppm | BDL | 0.01 | BDL | BDL | BDL |
| After filtration | | Mg | ppm | 6.11 | 0.19 | 0.3 | 0.22 | 0.2 |
| | | Mn | ppm | 0.07 | BDL | BDL | BDL | BDL |
| | | Na | ppm | BDL | 5.19 | 2.2 | 1.5 | 0.1 |
| | | Ni | ppm | 0.11 | BDL | BDL | BDL | BDL |
| | | Pb | ppm | 2.39 | BDL | BDL | BDL | BDL |
| | | Sr | ppm | 0.02 | BDL | BDL | BDL | BDL |
| | | V | ppm | BDL | BDL | BDL | BDL | BDL |
| | | Zn | ppm | 1.23 | 0.61 | 0.7 | 2.5 | 2.5 |
| | | Total | ppm | 50.14 | 15.61 | 12.86 | 9.22 | 13.2 |
| | Particle amount | ≥5.0 µm | pieces/mL | 1.9 | 0.2 | 0.1 | 0.7 | 0.8 |
| | | 5.0-1.0 µm | pieces/mL | 13.0 | 2.4 | 2.7 | 1.1 | 2.1 |
| | | 1.0-0.5 µm | pieces/mL | 43.8 | 5.4 | 5.9 | 7.9 | 5.2 |
| | | 0.5-0.3 µm | pieces/mL | 85.5 | 3.0 | 4.2 | 5.5 | 2.1 |
| | Dielectric strength | | kV | 60 | 67 | 65 | 70 | 69 |

| | | | | | | | | |
|---|---|---|---|---|---|---|---|---|
| *In Table, BDL represents below detection limits. | | | | | | | | |

## Claims

1. A composition for heat transfer fluid, the composition comprising:
a hexafluoropropene trimer represented by C₉F₁₈; and
(i) CₘF₂ₘ and/or CₙF₍₂ₙ₋₂₎ where m is an integer of 4 to 12 except 9 and n is an integer of 4 to 12, (ii) water where a content of the water relative to a total amount of the hexafluoropropene trimer represented by C₉F₁₈ regarded as 100 parts by mass is from 0.0001 to 0.1 parts by mass, and/or (iii) fluoride ions where a content of the fluoride ions relative to the total amount of the hexafluoropropene trimer represented by C₉F₁₈ regarded as 100 parts by mass is from 0.0000001 to 5 parts by mass.

2. The composition for heat transfer fluid according to claim 1,
wherein the hexafluoropropene trimer includes at least one selected from the group consisting of compounds represented by formulae (I) to (III) below.

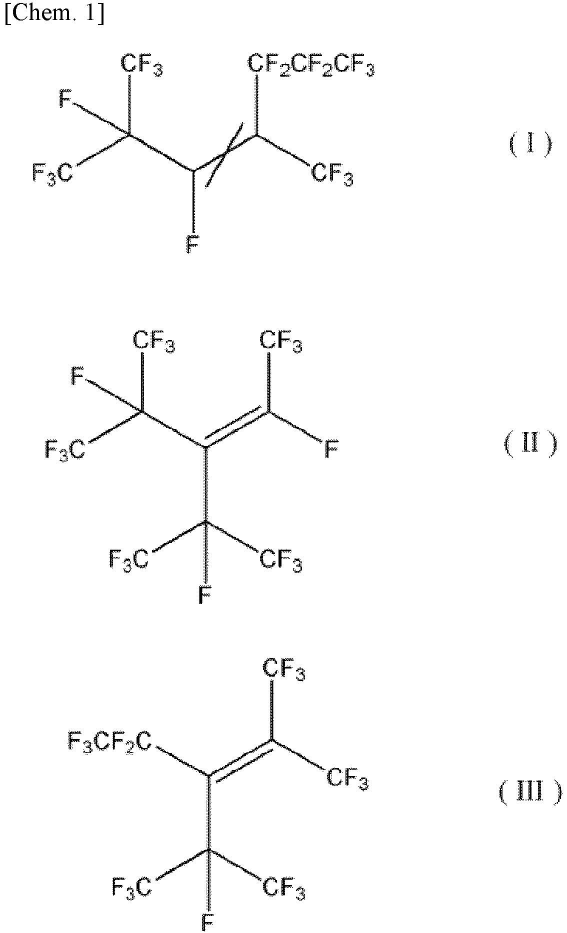

3. The composition for heat transfer fluid according to claim 2,
wherein a content of the compound represented by the formula (I) relative to the total amount of the hexafluoropropene trimer is 85 mass% or more.

4. The composition for heat transfer fluid according to claim 2,
wherein a content of the compound represented by the formula (I) relative to the total amount of the hexafluoropropene trimer is less than 85 mass%.

5. The composition for heat transfer fluid according to claim 2,
wherein a content of the compound represented by the formula (I) relative to the total amount of the hexafluoropropene trimer is not less than 50 mass% and less than 85 mass%.

6. The composition for heat transfer fluid according to claim 1, comprising the hexafluoropropene trimer represented by C₉F₁₈ and (i) CₘF₂ₘ and/or CₙF₍₂ₙ₋₂₎ where m is an integer of 4 to 12 except 9 and n is an integer of 4 to 12.

7. The composition for heat transfer fluid according to claim 1,
wherein m is an integer of 6 to 11 except 9 and n is an integer of 6 to 11.

8. The composition for heat transfer fluid according to claim 1,
wherein a content of CₘF₂ₘ and/or CₙF₍₂ₙ₋₂₎ relative to the total amount of the hexafluoropropene trimer represented by C₉F₁₈ regarded as 100 parts by mass is from 0.0001 to 10 parts by mass.

9. The composition for heat transfer fluid according to claim 1, comprising the hexafluoropropene trimer represented by C₉F₁₈ and (ii) water.

10. The composition for heat transfer fluid according to claim 1, comprising the hexafluoropropene trimer represented by C₉F₁₈ and (iii) fluoride ions.

11. The composition for heat transfer fluid according to claim 1, further comprising a hexafluoropropene tetramer represented by C₁₂F₂₄, wherein a content of the hexafluoropropene trimer relative to a total amount of the hexafluoropropene trimer and the hexafluoropropene tetramer is 80 mass% or more.

12. The composition for heat transfer fluid according to claim 11,
wherein the content of the hexafluoropropene trimer relative to the total amount of the hexafluoropropene trimer and the hexafluoropropene tetramer is from 90 mass% to 99.99 mass%.

13. The composition for heat transfer fluid according to claim 11,
wherein the hexafluoropropene tetramer includes 1,1,1,2,5,6,6,6-octafluoro-2,3,5-tris(trifluoromethyl)-4-(perfluoropropyl-2-yl)-3-hexene.

14. The composition for heat transfer fluid according to claim 1, further comprising a conductive substance, wherein a content of the conductive substance is 100 ppm by mass or less.

15. The composition for heat transfer fluid according to claim 1, further comprising a conductive substance, wherein a content of insoluble matter of 5 µm or more is 10 pieces/mL or less.

16. A heat transfer fluid comprising the composition for heat transfer fluid according to claim 1 or 2.

17. Use of the composition for heat transfer fluid according to claim 1 or 2 as a heat transfer fluid.

18. A heat transfer apparatus comprising:
a device; and
a mechanism for transferring heat to the device or from the device, wherein the mechanism comprises the composition for heat transfer fluid according to claim 1 or 2.

19. A heat transfer method comprising:
preparing a device; and
transferring heat to the device or from the device with the use of the composition for heat transfer fluid according to claim 1 or 2.
